# EUROPÄISCHE PATENTSCHRIFT

(11) **EP 2 461 844 B1**
(45) Veröffentlichungstag und Bekanntmachung des Hinweises auf die Patenterteilung: **11.12.2013**
(21) Anmeldenummer: 10747579.0
(22) Anmeldetag: 31.07.2010
(51) Int. Cl.: A61M 1/16

(54) **VORRICHTUNG UND VERFAHREN ZUR ERKENNUNG VON BLUT ODER BLUTBESTANDTEILEN IM FLÜSSIGKEITSSYSTEM EINER VORRICHTUNG ZUR EXTRAKORPORALEN BLUTBEHANDLUNG**
DEVICE AND METHOD FOR DETECTING BLOOD OR BLOOD CONSTITUENTS IN THE LIQUID SYSTEM OF A DEVICE FOR EXTRACORPOREAL BLOOD TREATMENT
DISPOSITIF ET PROCÉDÉ POUR DÉTECTER DU SANG OU DES CONSTITUANTS DU SANG DANS LE SYSTÈME LIQUIDE D'UN DISPOSITIF DE TRAITEMENT EXTRACORPOREL DU SANG

(30) Priorität: 04.08.2009 DE 102009036044
(43) Veröffentlichungstag der Anmeldung: 13.06.2012
(73) Patentinhaber: Fresenius Medical Care Deutschland GmbH, 61352 Bad Homburg v.d.H. (DE)
(72) Erfinder: BADO, Itka, 60385 Frankfurt (DE); SCHEUNERT, Peter, 61381 Friedrichsdorf (DE)
(74) Vertreter: Oppermann, Frank
(86) Internationale Anmeldenummer: PCT/EP2010/004697
(87) Internationale Veröffentlichungsnummer: WO 2011/015321

(56) Entgegenhaltungen:
- DE-U1-202004 003 335
- US-A1- 2003 210 390
- US-A1- 2005 045 540

## Beschreibung

Die Erfindung betrifft eine Vorrichtung und ein Verfahren zur Erkennung von Blut oder Blutbestandteilen im Flüssigkeitssystem einer Vorrichtung zur extrakorporalen Blutbehandlung, die einen durch eine semipermeable Membran in eine erste Kammer und eine zweite Kammer unterteilten Dialysator oder Filter aufweist, wobei die erste Kammer Teil des extrakorporalen Blutkreislaufs und die zweite Kammer Teil des Flüssigkeitssystems der extrakorporalen Blutbehandlungsvorrichtung ist.

Es sind verschiedene Verfahren zur extrakorporalen Blutbehandlung bekannt. Bei der Hämodialyse (HD) wird das Blut des Patienten in einem extrakorporalen Blutkreislauf gereinigt, der einen Dialysator umfasst. Der Dialysator weist eine Blutkammer und eine Dialysierflüssigkeitskammer auf, die von einer semipermeablen Membran getrennt sind. , Während bei der Hämodialyse (HD) die Dialysierflüssigkeitskammer von Dialysierflüssigkeit durchflossen wird, wobei Substanzen aufgrund der Diffusion zwischen der Dialysierflüssigkeit und dem Blut durch die Membran transportiert werden, wird bei der Hämofiltration (HF) die Dialysierflüssigkeitskammer des Dialysators nicht von Dialysierflüssigkeit durchströmt. Bei der Hämofiltration (HF) werden bestimmte Substanzen aufgrund von Konvektion durch die Membran des Filters effektiv entfernt. Eine Kombination aus beiden Verfahren ist die Hämodiafiltration (HDF).

Bei der Durchführung einer extrakorporalen Blutbehandlung besteht grundsätzlich das Risiko einer Ruptur der semipermeablen Membran des Dialysators oder Filters. Auch kann sich die Vergussmasse des Dialysators oder Filters ablösen. Im Falle eines Defekts des Dialysators oder Filters gelangt Blut aus dem extrakorporalen Blutkreislauf in das Flüssigkeitssystem der Blutbehandlungsvorrichtung. Daher wird bei den bekannten Blutbehandlungsvorrichtungen der Eintritt von Blut in das Flüssigkeitssystem infolge eines Defekts des Dialysators oder Filters überwacht. Die Erkennung von Blut im Flüssigkeitssystem erfolgt nach dem Stand der Technik mit einem optischen Messverfahren, wobei die Abnahme der Intensität von durch die Dialysierflüssigkeit hindurchtretenden Licht ausgewertet wird. Beim Eintritt von Blut in die Dialysierflüssigkeit ändert sich die Intensität des aus der Dialysierflüssigkeit austretenden Lichts, wobei die Intentsitätsänderung von der Wellenlänge des Lichts abhängig ist. Mit den bekannten Verfahren kann ein Bluteintritt in das Flüssigkeitssystem sicher erkannt werden.

Aus der US 2003/0210390 A1 ist eine Vorrichtung zur Erkennung von Blut im Flüssigkeitssystem einer Dialysevorrichtung aufgrund eines Defekts der Membran des Dialysators bekannt. Der bekannte Blutleckdetektor weist ein Gehäuse auf, in das eine transparente Schlauchleitung eingelegt wird. In dem Gehäuse befindet ein Lichtsender und ein Lichtempfänger auf beiden Seiten der eingelegten Schlauchleitung.

Neben dem Bluteintritt in das Flüssigkeitssystem infolge eines Defekts des Dialysators oder Filters, beispielsweise aufgrund einer Membranruptur oder einer Vergussmassenablösung, kann bei einer extrakorporalen Blutbehandlung auch freies Hämoglobin oder seine Bestandteile in die Dialysierflüssigkeit aufgrund einer Hämolyse gelangen. Als Hämolyse bezeichnet man die Auflösung (Zerstörung) der Erythrozyten (roten Blutkörperchen) des Bluts. Die Erythrozyten bestehen maßgeblich aus dem Sauerstoff bindenden Protein Hämoglobin, das den Erythrozyten und somit auch dem Blut die rote Farbe verleiht. Beim Auftreten von Hämolyse wird das Hämoglobin freigesetzt.

Eine Hämolyse kann bei einer extrakorporalen Blutbehandlung beispielsweise aufgrund einer mechanischen Beanspruchung des Bluts durch Scherströmungen auftreten. Derartige Scherströmungen treten unter anderem dann auf, wenn eine Blut führende Schlauchleitung des Schlauchleitungssystems der Blutbehandlungsvorrichtung abgeknickt wird. Eine Hämolyse kann aber auch systemisch durch den Patienten bedingt sein.

Hämoglobin kann bei der extrakorporalen Blutbehandlung von der Blutseite des Dialysators durch die semipermeable Membran auf die Dialysatseite diffundieren. Daher kann das Hämoglobin mit den nach dem Stand der Technik bekannten optischen Messverfahren im Blut nachgewiesen werden.

Nachteilig ist, dass mit den bekannten optischen Messverfahren zwischen einem Eintritt von Blut infolge eines Defekts des Dialysators oder Filters oder dem Eintritt von Hämoglobin als Blutbestandteil infolge einer Hämolyse nicht unterschieden werden kann. Bei einem Defekt des Dialysators oder Filters oder einer Hämolyse sind aber unterschiedliche Maßnahmen einzuleiten. So muss beispielsweise bei einer Membranruptur der Dialysator ausgetauscht werden, während bei einer Hämolyse der Anwender darauf hingewiesen werden muss, geeignete Gegenmaßnahem einzuleiten, beispielsweise das Schlauchsystem zu wechseln oder zumindest von der Knickstelle zu befreien.

Die im Allgemeinen bei der extrakorporalen Blutbehandlung eingesetzten Überwachungsvorrichtungen zur Erkennung eines Bluteintritts oder einer Hämolyse beruhen auf der spektroskopischen Auswertung des roten und grünen Anteils des Lichts. In diesem Wellenlängenbereich kann aber nicht zwischen Blut und Hämoglobin unterschieden werden.

Die US 2007/0259436 A1 beschreibt ein Verfahren zum Nachweis von Hämoglobin in Blut, bei dem Licht mit einer Wellenlänge von 390 bis 460 nm eine Probe durchtritt. Dabei wird die Änderung der Lichtintensität für zwei oder mehr Wellenlängen bestimmt.

Ein Verfahren zur Erkennung einer Hämolyse, bei dem die Abnahme der Intensität des Blauanteils von Licht Berücksichtigung findet, ist auch aus der JP 62000838A bekannt.

Der Erfindung liegt die Aufgabe zu Grunde, eine Vorrichtung bereitzustellen, die bei einem Eintritt von Blut oder Blutbestandteilen in das Flüssigkeitssystem der extrakorporalen Blutbehandlungsvorrichtung erlaubt, eine Entscheidung über die Einleitung gezielter Gegenmaßnahmen zu treffen. Darüber hinaus ist eine Aufgabe der Erfindung, ein Verfahren anzugeben, das ermöglicht, eine Entscheidung zur Einleitung gezielter Gegenmaßnahmen beim Eintritt von Blut oder Blutbestandteilen in das Flüssigkeitssystem der Blutbehandlungsvorrichtung zu treffen.

Die Lösung dieser Aufgaben erfolgt erfindungsgemäß mit den Merkmalen der Patentansprüche 1 und 13. Vorteilhafte Ausführungsformen der Erfindung sind Gegenstand der Unteransprüche.

Die erfindungsgemäße Vorrichtung zur Erkennung von Blut oder Blutbestandteilen im Flüssigkeitssystem einer extrakorporalen Blutbehandlungsvorrichtung ist als eine Einrichtung zur Unterscheidung zwischen dem Eintritt von Blut in das Flüssigkeitssystem aufgrund eines Defekts des Dialysators oder Filters, beispielsweise einer Ruptur der semipermeablen Membran des Dialysators oder Filters, und dem Eintritt von Hämoglobin in das Flüssigkeitssystem aufgrund einer Hämolyse ausgebildet, wobei ein Defekt des Dialysators oder Filters oder eine Hämolyse auf der Grundlage der Änderung der Intensität von Licht festgestellt wird, das durch die im Flüssigkeitssystem befindliche Flüssigkeit hindurchtritt.

Es hat sich gezeigt, dass der Quotient aus der Intensität des Rot- oder Grünanteils des aus der Flüssigkeit austretenden Lichts und der Intensität des Blauanteils des austretenden Lichts zunimmt, wenn nicht hämolysiertes Blut in die Dialysierflüssigkeit gelangt. Auch nimmt der Quotient aus der Intensität des Rotanteils und Grünanteils des aus der Flüssigkeit austretenden Lichts zu, wenn nicht hämolysiertes Blut in die Dialysierflüssigkeit gelangt. Wenn hämolysiertes Blut in die Dialysierflüssigkeit gelangt, nimmt der Quotient aus der Intensität des Rotanteils und Blauanteils oder der Quotient aus der Intensität des Rotanteils und Grünanteils zu. Der Quotient des Grünanteils und Blauanteils ändert sich hingegen beim Eintritt von hämolysierten Blut nicht oder nicht wesentlich.

Eine Unterscheidung zwischen dem Eintritt von hämolysierten und nicht hämolysierten Blut kann dadurch getroffen werden, dass das Verhältnis von dem Rotanteil und dem Blauanteil des aus der Flüssigkeit austretenden Lichts ausgewertet wird. Es hat sich gezeigt, dass der Quotient aus dem Rotanteil und Blauanteil beim Eintritt von nicht hämolysierten Blut stärke zunimmt als beim Eintritt von hämolysierten Blut. Auch kann eine Unterscheidung zwischen dem Eintritt von hämolysierten und nicht hämolysierten Blut dadurch getroffen werden, dass das Verhältnis von dem Grünanteil und dem Blauanteil des aus der Flüssigkeit austretenden Lichts ausgewertet wird, da sich ein signifikanter Anstieg des Quotienten nur bei dem Eintritt von nicht hämolysierten Blut zeigt.

Eine bevorzugte Ausführungsform der Erfindung sieht vor, zwischen dem Eintritt von Blut in das Flüssigkeitssystem aufgrund eines Defekts des Dialyators oder Filters und dem Eintritt von Hämoglobin in das Flüssigkeitssystem aufgrund einer Hämolyse auf der Grundlage der Änderung der Intensität zumindest des Blauanteils des aus der Flüssigkeit austretenden Lichts zu unterscheiden. Neben dem Blauanteil können aber noch weitere Anteile des Lichts, beispielsweise der Rotanteil und/oder der Grünanteil ausgewertet werden. Es hat sich gezeigt, dass die Auswertung des Blauanteils eine sichere Differenzierung zwischen Blut und dem Blutbestandteil Hämoglobin in der Dialysierflüssigkeit erlaubt.

Eine weitere besonders bevorzugte Ausführungsform sieht die Auswertung sowohl des Blauanteils als auch des Rotanteils vor. Dabei werden die Intensität des Blauanteils des aus der Flüssigkeit austretenden Lichts einerseits und die Intensität des Rotanteils des aus der Flüssigkeit austretenden Lichts andererseits bestimmt. Die Intensität des Rotanteils wird mit der Intensität des Blauanteils verglichen. Da die Änderung der Lichtintensität sowohl des Blau- als auch Rotanteils ausgewertet und miteinander in Beziehung gesetzt werden, kann sicher zwischen Blut und Hämoglobin unterschieden werden. Die Intensität des in die Flüssigkeit eintretenden Lichts spielt für die erfindungsgemäße Vorrichtung und das erfindungsgemäße Verfahren keine Rolle, da zwei unterschiedliche Farbanteile ins Verhältnis gesetzt werden. Daher sind die erfindungsgemäße Vorrichtung und das erfindungsgemäße Verfahren unempfindlich gegenüber Schwankungen der Intensität der Lichtquelle.

Bei einer bevorzugten Ausführungsform wird der Quotient aus der Intensität des Rotanteils und der Intensität des Blauanteils mit einem vorgegebenen Grenzwert verglichen und/oder der Quotient aus der Intensität des Grünanteils und der Intensität des Blauanteils mit einem vorgegebenen Grenzwert verglichen Auf einen Eintritt von Hämoglobin infolge einer Hämolyse wird dann geschlossen, wenn der Quotient kleiner oder gleich dem vorgegebenen Grenzwert ist. Ist der Quotient hingegen größer als der vorgegebene Grenzwert, wird auf den Eintritt von Blut beispielsweise aufgrund einer Ruptur der Membran geschlossen. Als Grenzwert kann nur ein einziger Wert vorgegeben werden. Es ist aber auch möglich, als Grenzwert mehrere individuelle Werte für unterschiedliche Abfragen vorzugeben.

Eine besonders bevorzugte Ausführungsform sieht zunächst die Berechnung des Quotienten aus dem Rotanteil und dem Grünanteil des aus der Flüssigkeit austretenden Lichts zur Erkennung des Eintritts von hämolysierten Blut oder nicht hämolysierten Bluts in die Dialysierflüssigkeit vor, ohne jedoch zwischen dem Eintritt von hämolysierten Blut oder nicht hämolysierten Blut unterscheiden zu können. Erst wenn hämolysiertes Blut oder nicht hämolysiertes Blut erkannt ist, wird der Quotient aus dem Rot- und Blauanteil und/oder aus dem Grün- und Blauanteil zur Unterscheidung zwischen einem Defekt des Dialysators oder Filters oder einer Hämolyse bestimmt.

Bei einer bevorzugten Ausführungsform wird nicht nur die Intensität des aus der Flüssigkeit austretenden Lichts, sondern auch die Intensität des in die Flüssigkeit eintretenden Lichts überwacht. Die Überwachung der Intensität des in die Flüssigkeit eintretenden Lichts erlaubt die Erkennung eines Störfalls. Beispielesweise kann durch Vergleich der gemessenen Intensität des eintretenden Lichts mit einem vorgegebenen Grenzwert erkannt werden, ob die Mittel zum Senden von Licht, beispielsweise eine LED, defekt sind. Dies ist insbesondere dann von Interesse, wenn aus der Flüssigkeit austretendes Lichts nicht mehr empfangen wird. Denn der Grund hierfür kann sein, dass sich anstelle von Dialysierflüssigkeit Blut im Flüssigkeitssystem befindet, was einen massiven Störfall darstellt, oder nur die Lichtquelle defekt ist.

Es ist aber auch möglich, mit der gemessenen Intensität des in die Flüssigkeit eintretenden Lichts eine Steuerung oder Regelung vorzusehen, um die Lichtintensität der Lichtquelle unabhängig von den Umgebungsbedingungen, beispielsweise der Temperatur, konstant zu halten.

Die erfindungsgemäße Vorrichtung verfügt über Mittel zum Emittieren von Licht, das in die im Flüssigkeitssystem befindliche Flüssigkeit eintritt, und Mittel zum Empfangen von Licht, das aus der im Flüssigkeitssystem befindlichen Flüssigkeit austritt. Darüber hinaus weist die Vorrichtung eine Auswerteinheit zum Auswerten der Intensität des in die Flüssigkeit eintretenden und aus der Flüssigkeit austretenden Lichts auf.

Bei einer bevorzugten Ausführungsform sind die Mittel zum Emittieren von Licht eine Lichtquelle, die weißes Licht emittiert, beispielsweise eine weißes Licht emittierende LED oder eine weißes Licht emittierende RGB-LED, während die Mittel zum Empfangen von Licht ein Lichtsensor sind, der unterschiedliche Farbanteile des Lichts empfängt. Diese bevorzugte Ausführungsform hat den Vorteil, dass die Messapparatur einen einfacheren Aufbau als eine Messapparatur hat, bei der mehrere Lichtquellen vorgesehen sind, beispielsweise eine blaue, rote und grüne Lichtquelle, und mehrere Lichtsensoren vorgesehen sind, die beispielsweise blaues, rotes und grünes Licht empfangen. Darüber hinaus erweist sich die Verwendung einer Lichtquelle, die unspezifisches Licht (Weißlicht) emittiert, als vorteilhaft, da das Messergebnis weniger von Verschiebungen des Spektrums des Lichts beeinflusst werden kann, das von einzelnen Lichtquellen mit einem schmalen Wellenlängenband emittiert wird.

Eine weitere besonders bevorzugte Ausführungsform sieht eine Anpassung des vorgegebenen Grenzwerts oder der vorgegebenen Grenzwerte an den Patienten vor. Dadurch ist es möglich, auch bei Patienten, die an einer systemischen Hämolyse leiden, zwischen dem Eintritt von Blut oder Hämoglobin in das Flüssigkeitssystem zu unterscheiden. Bei einem krankheitsbedingten höheren Anteil von Hämoglobin im Blut (systemische Hämolyse) kann der vorgegebene Grenzwert entsprechend angepasst werden, so dass nicht allein schon wegen des erhöhten Anteils von Hämoglobin im Blut aufgrund einer systemischen Hämolyse auf den Eintritt von Hämoglobin in die Dialysierflüssigkeit geschlossen wird, der auf eine Schädigung des Blutes beispielsweise durch eine abgeknickte Blut führende Schlauchleitung zurückzuführen ist. Beispielsweise kann anhand der Patientendaten ein Satz von angepassten Grenzwerten ermittelt und vorgegeben werden.

Die Anpassung des vorgegebenen Grenzwertes oder der Grenzwerte erfolgt vorzugsweise auf der Grundlage von patientenspezifischen Daten, die vorzugsweise auf einer Eingabeeinheit eingegeben werden. Es ist aber auch möglich, dass der vorgegebene Grenzwert in Abhängigkeit von patientenspezifischen Daten ermittelt wird, die auf der Grundlage einer Referenzmessung gewonnen werden. Beispielsweise ist es möglich, vor Beginn oder zu Beginn oder während der Blutbehandlung eine Referenzmessung zu einem Zeitpunkt vorzusehen, bei dem von einer Hämolyse beispielsweise aufgrund des Abknickens einer Schlauchleitung nicht ausgegangen wird. Abweichungen von diesem Grenzwert lassen dann auf den Eintritt von Hämoglobin aufgrund einer Blutschädigung schließen.

Wenn der Eintritt von Blut und / oder Hämoglobin festgestellt wird, kann ein optisches und / oder akustisches und / oder taktiles Signal gegeben werden, um geeignete Gegenmaßnahmen einleiten zu können. Beispielsweise kann die Blutbehandlung beim Eintritt von Blut sofort unterbrochen werden. Es ist auch möglich, dass ein Steuersignal für einen Eingriff in die Maschinensteuerung erzeugt wird, um Gegenmaßnahmen automatisch einleiten zu können.

Das Ergebnis der Überwachung wird vorzugsweise auf einem Bildschirm angezeigt, bei dem es sich um einen sogenannten Touchscreen handeln kann. Auf dem Monitor können neben der Art der auftretenden Komplikationen, d.h. Membranruptur oder Hämolyse, die empfohlenen Gegenmaßnahmen, beispielsweise durch einen entprechenden Text und / oder entsprechende Symbole angezeigt werden. Beispielsweise kann zum Austausch des Schlauchleitungssystems oder des Dialysators aufgefordert werden.

Im Folgenden werden Ausführungsbeispiele der Erfindung unter Bezugnahme auf die Zeichnungen näher erläutert.

Es zeigen:
- Fig. 1: eine extrakorporale Blutbehandlungsvorrichtung zusammen mit einer Vorrichtung zur Erkennung von Blut oder Blutbestandteilen im Flüssigkeitssystem der Blutbehandlungsvorrichtung in stark vereinfachter schematischer Darstellung,
- Fig. 2: eine schematische Darstellung des Messfeldes des Lichtsensors der Vorrichtung zur Erkennung von Blut oder Blutbestandteilen,
- Fig. 3: eine schematische Darstellung der relativen Empfindlichkeit des Lichtsensors in Abhängigkeit von der Wellenlänge des Lichts,
- Fig. 4: eine schematische Darstellung der Extinktion als Funktion der Wellenlänge für den Fall des Eintritts von Blut oder von Hämoglobin in die Dialysierflüssigkeit,
- Fig. 5: die Veränderung der Signalstärke des Lichtsensors für die einzelnen Farbanteile des Lichts vor und nach dem Eintritt von Hämoglobin in die Dialysierflüssigkeit und
- Fig. 6: die Veränderung der Signalstärke des Lichtsensors für die einzelnen Farbanteile vor und nach dem Eintritt von Blut in die Dialysierflüssigkeit.

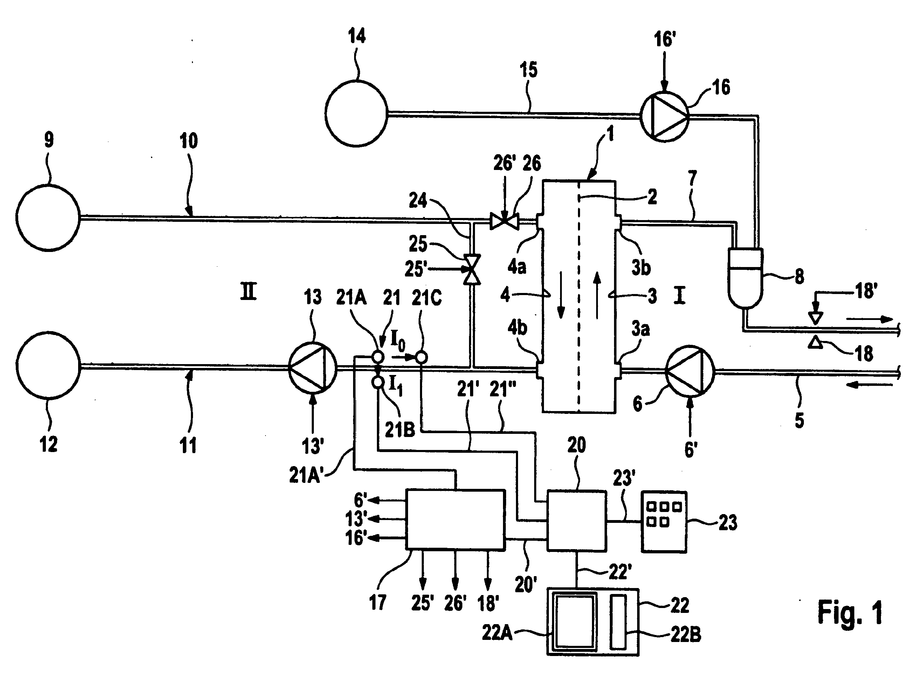

Fig. 1 zeigt in stark vereinfachter schematischer Darstellung ein Ausführungsbeispiel der für die Erfindung relevanten Komponenten einer extrakorporalen Blutbehandlungsvorrichtung, die als Hämodialysevorrichtung und / oder Hämofiltrationsvorrichtung betrieben werden kann. Daher wird die extrakorporale Blutbehandlungsvorrichtung nachfolgend auch als Hämodiafiltrationsvorrichtung bezeichnet.

Die Hämodiafiltrationsvorrichtung weist einen Dialysator oder Filter 1 auf, der durch eine semipermeable Membran 2 in eine Blutkammer 3 und eine Dialysierflüssigkeitskammer 4 getrennt ist. Der Einlass 3a der Blutkammer ist mit einem Ende einer arteriellen Blutzuführleitung 5 verbunden, in die eine Blutpumpe 6 geschaltet ist, während der Auslass 3b der Blutkammer mit einem Ende einer venösen Blutrückführleitung 7 verbunden ist, in die eine Tropfkammer 8 geschaltet ist. An den anderen Enden der arteriellen und venösen Blutleitung 5, 7 befinden sich die nicht dargestellten arteriellen und venösen Kanülen zum Anschluss an den Patienten. Dieser Teil des Flüssigkeitssystems stellt den extrakorporalen Blutkreislauf I der Hämodiafiltrationsvorrichtung dar. Bei den Blutleitungen 5, 7 handelt es sich um Schlauchleitungen eines zur einmaligen Verwendung bestimmten Schlauchleitungssystems, das in die Blutbehandlungsvorrichtung eingelegt wird.

Das Flüssigkeitssystem II der Hämodiafiltrationsvorrichtung umfasst eine Einrichtung 9 zur Bereitstellung frischer Dialysierflüssigkeit, die über eine Dialysierflüssigkeitszuführleitung 10 mit dem Einlass 4a der Dialysierflüssigkeitskammer 4 des Dialysators 1 oder Filters verbunden ist. Von dem Auslass 4b der Dialysierflüssigkeitskammer 4 des Dialysators 1 oder Filters geht eine Dialysierflüssigkeitsrückführleitung 11 ab, die zu einem Auslass 12 führt. Zum Fördern der Dialysierflüssigkeit dient eine Dialysierflüssigkeitspumpe 13, die in die Dialysierflüssigkeitsrückführleitung geschaltet ist.

Darüber hinaus verfügt die Hämodiafiltrationsvorrichtung über eine Substituatquelle 14, von der eine Substituatleitung 15, in die eine Substituatpumpe 16 geschaltet ist, zu der venösen Tropfkammer 8 führt. Mit der Substituatpumpe kann dem extrakorporalen Blutkreislauf 1 eine vorgegebene Menge an Substitutionsflüssigkeit aus der Substituatquelle zugeführt werden, wenn dem extrakorporalen Blutkreislauf I über den Dialysator 1 Flüssigkeit entzogen wird.

Die Hämodiafiltrationsvorrichtung umfasst weiterhin eine zentrale Steuer- und Recheneinheit 17, die über Steuerleitungen 6', 13', 16' mit der Blutpumpe 6, der Dialysierflüssigkeitspumpe 13 und der Substituatpumpe 16 verbunden ist. Die Steuer- und Recheneinheit 17 sendet an die einzelnen Komponenten Steuerbefehle und empfängt von den Komponenten Daten über deren Betriebszustände.

Nachfolgend wird die erfindungsgemäße Vorrichtung zur Erkennung von Blut oder Blutbestandteilen im Flüssigkeitssystem der extrakorporalen Blutbehandlungsvorrichtung beschrieben. Die erfindungsgemäße Vorrichtung zur Erkennung von Blut oder Blutbestandteilen kann eine selbständige Einheit bilden oder Bestandteil der Blutbehandlungsvorrichtung sein. Bei dem vorliegenden Ausführungsbeispiel ist die erfindungsgemäße Vorrichtung Bestandteil der Blutbehandlungsvorrichtung.

Die erfindungsgemäße Vorrichtung kann mit Ausnahme der Messanordnung von den Teilen Gebrauch machen, die bereits in den bekannten extrakorporalen Blutbehandlungsvorrichtungen vorhanden sind. Beispielsweise kann die erfindungsgemäße Vorrichtung von der zentralen Steuer- und Recheneinheit der Blutbehandlungsvorrichtung Gebrauch machen, um die gewonnenen Messdaten auszuwerten. Auch kann die erfindungsgemäße Vorrichtung die Anzeigeeinheit und die Eingabeeinheit der Blutbehandlungsvorrichtung verwenden. Es kann aber für die erfindungsgemäße Vorrichtung auch eine separate Auswerteinheit vorgesehen sein. Auch kann eine separate Anzeigeeinheit oder Eingabeeinheit vorhanden sein.

Die Vorrichtung zur Erkennung von Blut oder Hämoglobin verfügt über eine Lichtquelle 21 A, beispielsweise eine LED, und einen Lichtsensor 21 B, die einander gegenüberliegend angeordnet sind. Lichtquelle 21A und Lichtsensor 21B bilden eine Messanordnung 21 zum Messen der Änderung der Intensität von Licht, das aus der im Flüssigkeitssystem II befindliche Flüssigkeit, d.h. Dialysierflüssigkeit austritt. Da die Dialysierflüssigkeitsleitungen 10, 11 transparente Schlauchleitungen sind, kann das Licht durch die Schlauchleitung in die Dialysierflüssigkeit eingekoppelt und aus der Dialysierflüssigkeit ausgekoppelt werden. Die Messanordnung 21 ist an der Dialysierflüssigkeitsrückführleitung 11, insbesondere in dem Abschnitt der Dialysierflüssigkeitsrückführleitung 11 stromab der Dialysierflüssigkeitskammer 4 des Dialysators 1 oder Filters und stromauf der Dialysierflüssigkeitspumpe 13 angeordnet. Die Messung kann sowohl an einer zur einmaligen Verwendung bestimmten Messkammer oder an einer fest installierten Messkammer der Schlauchleitung erfolgen. Als Messkammer kann auch eine Küvette Verwendung finden. Neben dem ausgangsseitigen Lichtsensor 21 B kann die Messanordnung noch einen eingangsseitigen Lichtsensor 21 C aufweise, der das mit der Lichtquelle 21A emittierte Licht misst.

Für den Fall einer Ruptur der semipermeablen Membran 2 des Dialysators 1 oder Filters tritt sowohl beim Betrieb der Hämodiafiltrationsvorrichtung als Hämodialysevorrichtung als auch Hämofiltrationsvorrichtung Blut aus dem extrakorporalen Blutkreislauf I in die Dialysierflüssigkeitskammer 4 des Dialysators 1 oder Filters ein. Das nicht hämolysierte Blut kann dann mit der Messanordnung 21 in der Dialysierflüssigkeit nachgewiesen werden, die im Dialysierflüssigkeitssystem II strömt. Wenn die Hämodiafiltrationsvorrichtung nur als Hämofiltrationsvorrichtung betrieben wird, kann das Blut in dem Filtrat nachgewiesen werden, das der Dialysierflüssigkeitskammer 4 entzogen wird. Für den Fall einer Hämolyse infolge des Abknickens der arteriellen oder venösen Blutleitung 5, 7 oder einer systemisch bedingten Hämolyse gelangt Hämoglobin oder hämolysiertes Blut durch die intakte semipermeable Membran 2 des Dialysators 1 oder Filters in die Dialysierflüssigkeitskammer 4, welches ebenfalls mit der Messanordnung 21 nachgewiesen werden kann.

Stromab der Tropfkammer 8 befindet sich an der venösen Blutleitung 7 ein Absperrorgan 18 zum Absperren der Blutleitung 7. Das Absperrorgan 18, insbesondere eine elektromagnetisch betätigbare Schlauchklemme, ist über eine Steuerleitung 18' mit der Steuer- und Recheneinheit 17 verbunden.

Die Vorrichtung zur Erkennung von Blut oder Blutbestandteilen weist eine Auswerteinheit 20 auf, die über eine Datenleitung 20' mit der zentralen Steuer- und Recheneinheit 17 der Hämodiafiltrationsvorrichtung verbunden ist. Die Auswerteinheit 20 kann aber auch Bestandteil der Steuer- und Recheneinheit 17 sein. Die Auswerteinheit empfängt über eine Datenleitung 21' die Signale des ausgangsseitigen Lichtsensors 21 B und über eine Datenleitung 21 " die Signale des eingangsseitigen Lichtsensors 21C der Messanordnung 21. Die Auswerteinheit 20 kann eine Regeleinheit aufweisen, die in Abhängigkeit von der mit dem ausgangsseitigen Lichtsensors 2 1 B gemessenen Intensität des von der Lichtquelle 21 A emittierten Lichts die Lichtquelle derart ansteuert, das die Lichtintensität unabhängig von den Umgebungsbedingungen konstant bleibt.

Das Ergebnis der Überwachung wird mit einer Signaleinheit 22 signalisiert, die über eine Datenleitung 22' mit der Auswerteinheit 20 verbunden ist. Die Signaleinheit 22 weist einen Bildschirm 22A zum Anzeigen von Symbolen oder Text und einem Alarmgeber 22B zur Erzeugung eines akustischen oder taktilen Alarms auf. Auf einer Eingabeeinheit 23, die über eine Datenleitung 23' mit der Auswerteinheit 20 verbunden ist, können patientenspezifische Daten eingegeben werden. Bei der Eingabeeinheit 23 kann es sich auch um einen sogenannten Touchscreen handeln, der gleichsam als Bildschirm der Signaleinheit dient.

Die Lichtquelle 21A der Messanordnung 21 emittiert weißes Licht, das die Farbanteile Rot, Grün und Blau mit einer bestimmten Intensität I₀ enthält. Die Lichtquelle 21 A ist über eine Steuerleitung 21A' mit der zentralen Steuer- und Recheneinheit 17 verbunden, so dass die Intensität I₀ des Lichts von der Steuer- und Recheneinheit 17 vorgegeben werden kann. Der Lichtsensor 21B empfängt das durch die Dialysierflüssigkeit (Filtrat) hindurchtretende Licht der Lichtquelle 21 A. Bei dem Lichtsensor handelt es sich um einen Sensor, der die Intensität I₁ von drei Farbanteilen Rot, Grün und Blau (RGB) auswerten kann. Die lichtempfindliche Oberfläche des Sensors wird von einer Vielzahl identischer Fotodioden gebildet, die in Reihen und Spalten angeordnet sind. Jeder Fotodiode ist jeweils ein Farbfilter zugeordnet. Für die Auswertung des Rotanteils ist ein roter Farbfilter, für die Auswertung des Grünanteils ein grüner Farbfilter und für die Auswertung des Blauanteils des Lichts ein blauer Farbfilter vorgesehen.

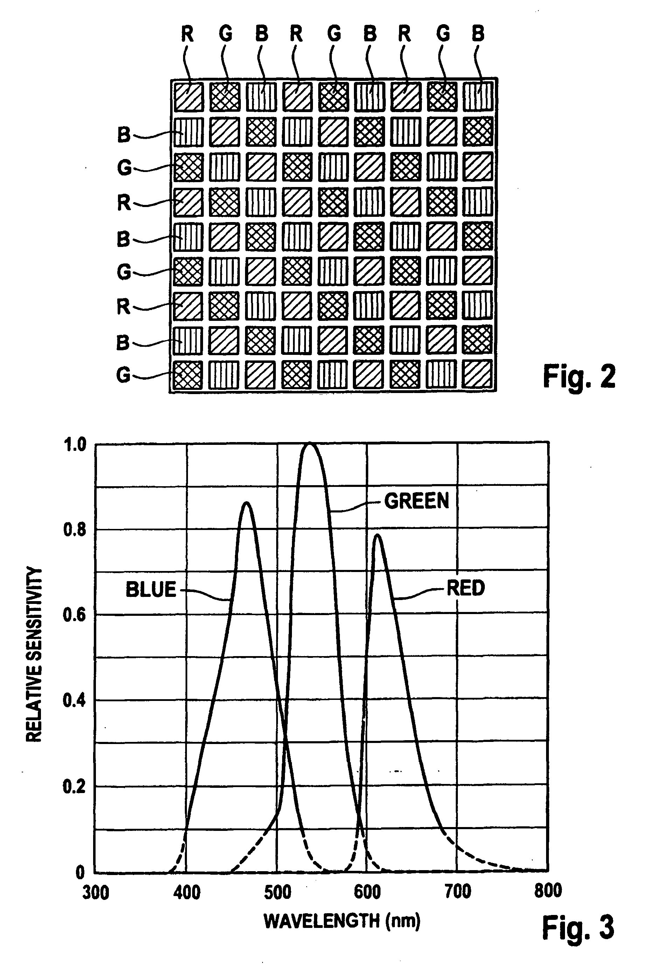

Fig. 2 zeigt die schachbrettartige Anordnung der Fotodioden mit den Farbfiltern. Die Farbfilter sind mit R (Rot), G (Grün) und B (Blau) gekennzeichnet. Der Lichtsensor 21 B liefert ein elektrisches Ausgangssignal, das Informationen zu der Intensität des Rot-, Grün- und Blauanteils des Lichts enthält. Fig. 3 zeigt in schematischer Darstellung die relative Empfindlichkeit des Sensors für die einzelnen Farbanteile Blau, Grün und Rot. Da der Lichtsensor nicht nur einen einzelnen Messpunkt, sondern ein Messfeld abbildet, liefert der Sensor 1 ein über die lichtempfindliche Oberfläche integriertes Ausgangssignal.

Bei dem Lichtsensor kann es sich beispielsweise um einen Sensor des Fabrikats "Programmable Color Light-To-Frequency Converter TCS230" der Firma TAOS (Texas Advanded Optoelectronic Solutions Inc., Piano, Texas, USA) oder um einen Sensor des Fabrikats "Digital Color Sensor S9706" der Firma Hamamatsu (Hamamatsu Photonics K.K., Japan) oder dergleichen handeln. Aufbau und Funktion der beiden Sensoren sind in den Datenblättern der Hersteller im Einzelnen beschrieben.

Nachfolgend wird die Funktionsweise der erfindungsgemäßen Vorrichtung zur Unterscheidung zwischen Blut und Hämoglobin in der Dialysierflüssigkeit (Filtrat) beschrieben.

Die Auswerteinheit 20 empfängt das die drei Farbanteile Rot, Grün und Blau enthaltende Ausgangssignal S des ausgangseitigen Lichtsensors 21B der Messanordnung 21. In der Auswerteinheit 20 wird das Ausgangssignal des Sensors in die drei Einzelsignale für die Farbbestandteile Rot, Grün und Blau (RGB) zerlegt. Der Betrag der Einzelsignale (Signalstärke) ist proportional der Intensität I₁ des aus der Dialysierflüssigkeit (Filtrat) austretenden Lichts. Die Intensität I₀ des in die Dialysierflüssigkeit (Filtrat) eintretenden Lichts wird von der Lichtquelle 21 A bestimmt und mit dem eingangsseitigen Lichtsensor 21C gemessen. Die Auswerteinheit 20 bestimmt nunmehr den Quotienten R/B aus der Intensität I₁ des aus der Flüssigkeit austretenden Rotanteils R des Lichts, d.h. dem Betrag des Einzelsignals für den Rotanteil, und der Intensität I₁ des aus der Flüssigkeit austretenden Blauanteils B des Lichts, d.h. dem Betrag des Einzelsignals für den Blauanteil. Darüber hinaus kann die Auswerteinheit den Quotienten R/G aus der Intensität I₁ des Rotanteils und des Grünanteils, d.h. den Quotienten der Einzelsignale des Rot- und Grünanteils berechnen. Ferner berechnet die Auswerteinheit den Quotienten G/B der Intensität I₁ des Grünanteils und des Blauanteils. Sämtliche Quotienten werden in einem internen Speicher der Auswerteinheit abgelegt. Die Messung kann in vorgegebenen Zeitabständen oder kontinuierlich erfolgen. Es ist auch möglich, zu bestimmten Zeitpunkten, beispielsweise vor der Behandlung, Referenzmessungen durchzuführen, um die aktuellen Werte mit den Referenzwerten vergleichen zu können. Die Referenzmessungen können auch zyklisch beispielsweise unter Verwendung eines den Dialysator oder Filter umgehenden Bypasses erfolgen.

Der Bypass zur Umgehung des Dialysators für die Referenzmessungen umfasst eine Bypass-Leitung 24, die von der Dialysierflüssigkeitszuführleitung 10 stromauf der Dialysierflüssigkeitskammer 4 abgeht und stromab der Dialysierflüssigkeitskammer zu der Dialysierflüssigkeitsabführleitung 11 führt. In der Bypass-Leitung 24 befindet sich ein Absperrorgan 25 zum Öffnen und Schließen der Bypassleitung und in dem Abschnitt der Dialysierflüssigkeitszuführleitung 10 zwischen der Bypass-Leitung 24 und der Dialysierflüssigkeitskammer 4 befindet sich ein Absperrorgan 26 zum Abtrennen der Dialysierflüssigkeitskammer. Die beiden Absperrorgane 25, 26 sind über Steuerleitungen 25', 26' mit der zentralen Steuer- und Recheneinheit 17 verbunden. Zur Umgehung des Dialysators 1 öffnet die Steuereinheit das Absperrorgan 24 und schließt das Absperrorgan 25. Ansonsten sind das Absperrorgan 24 geschlossen und das Absperrorgan 25 geöffnet.

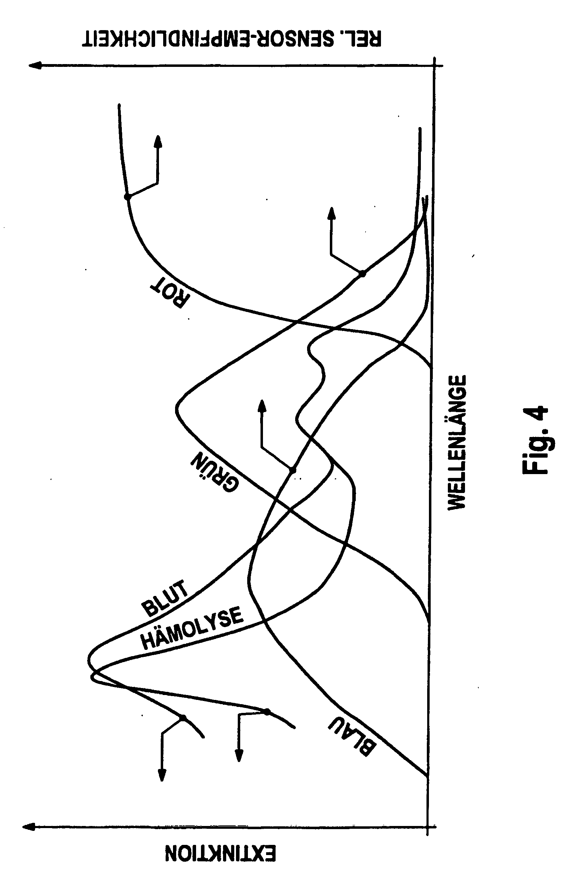

Fig. 4 zeigt schematisch die charakteristischen Verläufe der Extinktion für intaktes Blut im Dialysat einerseits und für hämolysiertes Blut im Dialysat andererseits sowie die relative Empfindlichkeit des Sensors in Abhängigkeit von der Wellenlänge. Man kann erkennen, dass im Bereich des blauen bis grünen Lichts beträchtliche Unterschiede für intaktes Blut und hämolysiertes Blut bestehen, weil das hämolysierte Blut freies Hämoglobin in das Dialysat einträgt. Im Bereich des grünen bis roten Lichts sind hingegen kaum bis keine Unterschiede zu erkennen. Der Graf der Extinktion für intaktes Blut im Dialysat unterscheidet sich unter anderem im Bereich des blauen Spektrums von dem Graf der Extinktion für das hämolysierte Blut im Dialysat.

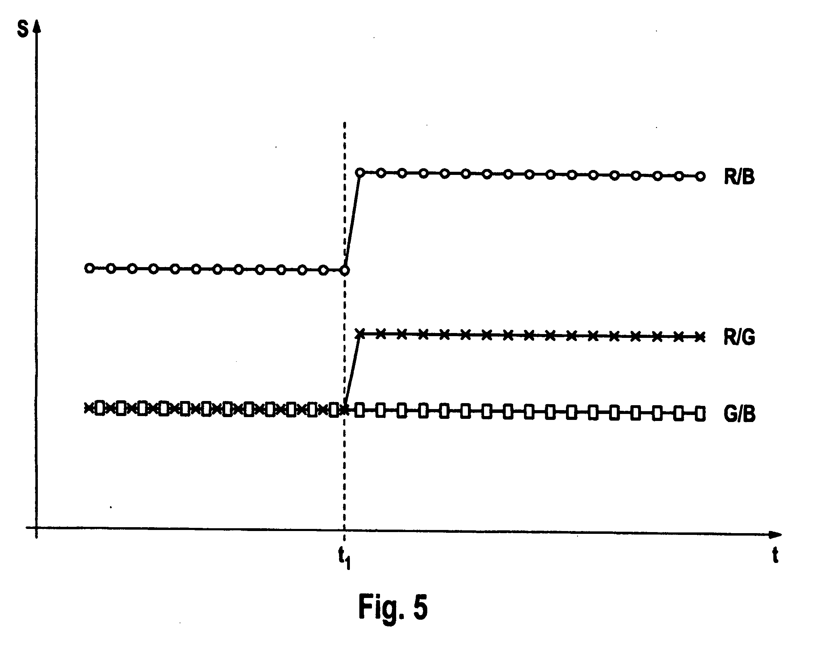

Fig. 5 zeigt den Betrag des Quotienten der Einzelsignale S des Farbsensors für die einzelnen Farbanteile des Lichts ROT / BLAU (R/B), ROT / GRÜN (R/G), GRÜN / BLAU (G/B) in Abhängigkeit von der Zeit vor dem Eintritt (t < t₁) und nach dem Eintritt (t > t₁) von hämolysiertem Blut in das Dialysat. Es zeigt sich, dass mit der Zugabe von Hämoglobin der Betrag des Quotienten aus dem Rot- und Blauanteil (ROT / BLAU) und der Betrag des Quotienten aus dem Rot- und Grünanteil (ROT / GRÜN) signifikant zunimmt, während sich der Betrag des Quotienten des Grün- und Blauanteils (GRÜN / BLAU) kaum oder nicht signifikant verändert.

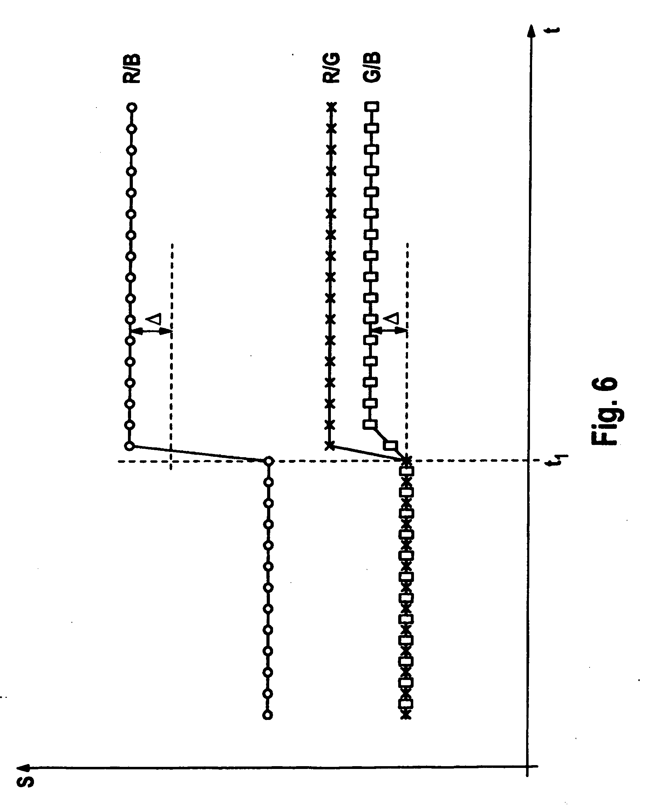

Fig. 6 zeigt den Fall des Eintritts von nicht hämolysiertem Blut in das Dialysat. Es zeigt sich, dass der Betrag sämtlicher Quotienten (ROT / BLAU, ROT / GRÜN und GRÜN / BLAU) mit dem Eintritt von nicht hämolysiertem Blut signifikant zunimmt. Ein Vergleich der Figuren 5 und 6 zeigt, dass der Eintritt von nicht hämolysiertem Blut (Fig. 6) allerdings zu einem stärkeren Anstieg des Betrags des Quotienten aus dem Rot- und Blauanteil (ROT / BLAU) führt, als der Eintritt von hämolysiertem Blut (Fig. 5). Während bei dem Eintritt von hämolysierten Blut der Betrag des Quotienten von dem Grün- und Blauanteil (GRÜN / BLAU) nahezu unverändert bleibt (Fig. 5), nimmt der Betrag des Quotienten von dem Grün- und Blauanteil (GRÜN / BLAU) bei dem Eintritt von nicht hämolysiertem Blut zu. Bei dem Betrag des Quotienten aus dem Rot- und Grünanteil (ROT / GRÜN) ist hingegen in den Figuren 5 und 6 kein signifikanter Unterschied erkennbar.

Der Unterschied der Zunahme des Betrags des Quotienten aus dem Rot- und Blauanteil (ROT / BLAU) bei hämolysiertem Blut und nicht hämolysiertem Blut und der Unterschied zwischen dem Betrag des Quotienten aus dem Grün- und Blauanteil (GRÜN / BLAU) bei hämolysiertem und nicht hämolysierten Blut ist in Figur 6 mit Δ bezeichnet.

Bei einer ersten Ausführungsform vergleicht die Auswerteinheit 20 den Quotienten aus dem Rot- und Blauanteil (ROT / BLAU) mit einem vorgegebenen Grenzwert, der verfahrensabhängig verändert werden kann. Bei dieser Ausführungsform braucht nur der Quotient R/B des Rot- und Blauanteils berechnet zu werden, der mit dem vorgegebenen Grenzwert verglichen wird. Wenn der Quotient von dem Rot- und Blauanteil (ROT / BLAU) oberhalb des vorgegebenen Grenzwerts liegt, schließt die Auswerteinheit 20 auf den Eintritt von nicht hämolysiertem Blut beispielsweise aufgrund einer Membranruptur (Fig. 6), während die Auswerteinheit auf den Eintritt von hämolysiertem Blut beispielsweise in Folge einer Blutschädigung beispielsweise wegen einer abgeknickten Schlauchleitung oder einer systemisch bedingten Hämolyse schließt (Fig. 5), wenn der Quotient von dem Rot- und Blauanteil (ROT / BLAU) kleiner oder gleich dem vorgegebenen Grenzwert ist.

Bei einer alternativen Ausführungsform berechnet die Auswerteinheit 20 nicht den Quotienten des Rot- und Blauanteils, sondern den Quotienten des Grün- und Blauanteils. Die Auswerteinheit schließt auf den Eintritt von nicht hämolysiertem Blut (Fig. 6), wenn der Quotient von dem Grün- und Blauanteil (GRÜN / BLAU) größer als der vorgegebene Grenzwert ist, während die Auswerteinheit auf den Eintritt von hämolysiertem Blut schließt (Fig. 5), wenn der Quotient von dem Grün- und Blauanteil gleich oder kleiner als der vorgegebene Grenzwert ist.

Es ist auch möglich, dass beide Quotienten (ROT / BLAU) und (GRÜN / BLAU) bestimmt werden und statistisch ausgewertet werden. Beispielsweise ist es möglich, dass die Auswerteinheit nur auf den Eintritt von nicht hämolysiertem Blut schließt, wenn sowohl der Quotient des Rot- und Blauanteils als auch der Quotient des Grün- und Blauanteils oberhalb der vorgegebenen Grenzwerte liegen.

Grundsätzlich ist es aber auch möglich, nur den Blauanteil des Lichts auszuwerten, der weder mit dem Rotanteil noch den Grünanteil in Beziehung gesetzt wird.

Bei einer bevorzugten Ausführungsform bestimmt die Auswerteinheit 20 nicht nur den Quotienten aus dem Rot- und Blauanteil und/oder dem Grün- und Blauanteil, um zwischen dem Eintritt von hämolysierten und nicht hämolysierten Blut unterscheiden zu können, sondern auch den Quotienten aus dem Rot- und Grünanteils, der in dem Speicher abgelegt wird. Der Quotient aus dem Rot- und Grünanteil (ROT/GRÜN) wird in der Auswerteinheit 20 mit einem ersten vorgegebenen Grenzwert verglichen. Wenn der Quotient aus dem Rot- und Grünanteil (ROT/GRÜN) größer als der vorgegebene erste Grenzwert ist, schließt die Auswerteinheit auf den Eintritt von hämolysierten Blut oder nicht hämolysierten Blut in die Dialysierflüssigkeit. Ansonsten wird auf einen Störfall nicht geschlossen. Erst wenn die Auswerteineheit einen Störfall festgestellt hat, d.h. der Quotient aus dem Rot- und Grünanteil (ROT/GRÜN) größer als der vorgegebene erste Grenzwert ist, leitet die Auswerteinheit die oben beschriebenen Schritte ein, um zwischen dem Eintritt von hämolysierten Blut oder nicht hämolysierten Blut unterscheiden zu können. Die Auswerteinheit berechnet dann den Quotienten aus dem Rot- und Blauanteil (ROT/BLAU), der mit einem vorgegebenen zweiten Grenzwert verglichen wird, und/oder den Quotienten aus dem Grün- und Blauanteil (Grün/BLAU), der mit einem vorgegebenen dritten Grenzwert verglichen wird, wie oben beschrieben ist.

Die vorgegebenen Grenzwerte können in einem Speicher in der Auswerteinheit 20 abgelegt sein. Damit sind die vorgegebenen Grenzwerte fest vorgegeben. Bei einer alternativen Ausführungsform werden die vorgegebenen Grenzwerte an den Patienten angepasst. Hierzu verfügt die Auswerteinheit 20 über Mittel zum Anpassen des Grenzwertes an patientenspezifische Daten, die auf der Eingabeeinheit 23 eingegeben werden. Beispielsweise ist es möglich, für Patienten, die an einer systemischen Hämolyse leiden, einen höheren Grenzwert vorzugeben, als Patienten, bei denen das Hämoglobin im Blut nicht erhöht ist. Die Auswerteinheit nimmt also nach der Eingabe der patientenspezifischen Daten für einen Patienten, der an systemischer Hämolyse leidet, eine Korrektur des ansonsten vorgegebenen Grenzwertes mit einem bestimmten Korrekturfaktor vor.

Eine alternative Ausführungsform sieht die Ermittlung des Grenzwertes mit einer Referenzmessung vor, die vor oder zu Beginn oder auch während der Blutbehandlung zu einem Zeitpunkt vorgenommen wird, zu dem von einer Komplikation nicht ausgegangen wird. Mit dieser Referenzmessung werden die möglicherweise aufgrund einer systemischen Hämolyse erhöhten Hämoglobinwerte ermittelt, aus denen ein entsprechender Korrekturfaktor berechnet wird.

Wenn die Auswerteinheit den Eintritt von hämolysiertem Blut beispielsweise aufgrund des Abknickens einer Schlauchleitung oder den Eintritt von nicht hämolysiertem Blut beispielsweise aufgrund einer Membranruptur erkennt, wird das Ergebnis der Überprüfung mit der Signaleinheit 22 signalisiert. Beispielsweise wird mit dem Alarmgeber 22B ein akustischer und/oder optischer und/oder taktiler Alarm gegeben. Auf dem Bildschirm 22B der Signaleinheit 22 können optische Alarmsymbole angezeigt werden. Auch kann auf dem Bildschirm ein Hinweis auf eine gezielte Gegenmaßnahme gegeben werden. Beispielsweise kann auf dem Bildschirm angezeigt werden, dass das Schlauchsystem oder der Dialysator zu wechseln ist. Es ist auch möglich, auf dem Bildschirm darauf hinzuweisen, dass zwar Hämoglobin im Dialysat erkannt wird, das aber nicht auf eine Hämolyse in Folge einer Schädigung des Bluts im extrakorporalen Kreislauf, sondern auf eine systemische bedingte Hämolyse des Patienten zurückzuführen ist.

Neben der Signalisierung der Komplikationen der extrakorporalen Blutbehandlung ist es auch möglich, dass die Auswerteinheit ein Steuersignal zum Eingriff in die Maschinensteuerung erzeugt, das die zentrale Steuer- und Recheneinheit 17 der extrakorporalen Blutbehandlungsvorrichtung empfängt. Für den Fall, dass die Auswerteinheit eine Membranruptur detektiert, erzeugt die Auswerteinheit ein Steuersignal für die sofortige Unterbrechung der Blutbehandlung. Die zentrale Steuer- und Recheneinheit 17 unterbricht dann sofort die Blutförderung durch Schließen der Schlauchklemme 18 und Stoppen der Blutpumpe 6. Auf dem Bildschirm erscheint nunmehr eine Aufforderung zum Austausch des Dialysators 1 oder Filters, während ein akustischer Alarm mit dem Alarmgeber 22B gegeben wird.

## Patentansprüche

1. Vorrichtung zur Erkennung von Blut oder Blutbestandteilen im Flüssigkeitssystem einer Vorrichtung zur extrakorporalen Blutbehandlung, die einen durch eine semipermeable Membran (2) in eine erste Kammer (3) und eine zweite Kammer (4) unterteilten Dialysator (1) oder Filter aufweist, wobei die erste Kammer Teil eines extrakorporalen Blutkreislaufs (I) und die zweite Kammer Teil eines Flüssigkeitssystems (II) ist, mit
Mitteln (21 A) zum Emittieren von Licht, das in die im Flüssigkeitssystem befindliche Flüssigkeit eintritt, und Mitteln (21B) zum Empfangen von Licht, das aus der im Flüssigkeitssystem befindlichen Flüssigkeit austritt, und
einer Auswerteinheit (20), die Mittel zum Auswerten der Intensität I₁ des aus der Flüssigkeit austretenden Lichts aufweist,
**dadurch gekennzeichnet, dass**
die Vorrichtung zur Erkennung von Blut oder Blutbestandteilen im Flüssigkeitssystem als eine Einrichtung zur Unterscheidung zwischen dem Eintritt von Blut in das Flüssigkeitssystem aufgrund eines Defekts des Dialysators oder Filters oder des Eintritts von Hämoglobin in das Flüssigkeitssystem aufgrund einer Hämolyse auf der Grundlage der Änderung der Intensität I₁ des aus der Flüssigkeit austretenden Lichts ausgebildet ist.

2. Vorrichtung nach Anspruch 1, **dadurch gekennzeichnet, dass** die Einrichtung zur Erkennung von Blut oder Blutbestandteilen im Flüssigkeitssystem derart ausgebildet ist, dass zwischen dem Eintritt von Blut in das Flüssigkeitssystem aufgrund eines Defekts des Dialysators oder Filters oder des Eintritts von Hämoglobin in das Flüssigkeitssystem aufgrund einer Hämolyse zumindest auf der Grundlage der Änderung der Intensität I₁ des Blauanteils des aus der Flüssigkeit austretenden Lichts unterschieden wird.

3. Vorrichtung nach Anspruch 1 oder 2, **dadurch gekennzeichnet, dass** die Mittel (21) zum Emittieren von Licht und die Mittel (21) zum Empfangen von Licht als Mittel zum Emittieren und Empfangen von Licht ausgebildet sind, das zumindest einen Blauanteil und einen Rotanteil aufweist, und
dass die Auswerteinheit (20) derart ausgebildet ist, dass die Intensität I_{1/B} des Blauanteils des aus der Flüssigkeit austretenden Lichts und die Intensität I_{1/R} des Rotanteils des aus der Flüssigkeit austretenden Lichts bestimmt wird, und
dass die Auswerteinheit (20) derart ausgebildet ist, dass die Intensität I_{1/R} des Rotanteils mit der Intensität I_{1/B} des Blauanteils verglichen wird, wobei zwischen dem Eintritt von Blut in das Flüssigkeitssystem und einer Hämolyse auf der Grundlage des Vergleichs zwischen der Intensität des Rotanteils und der Intensität des Blauanteils unterschieden wird.

4. Vorrichtung nach Anspruch 3, **dadurch gekennzeichnet, dass** die Auswerteinheit (20) derart ausgebildet ist, dass der Quotient (R/B) aus der Intensität des Rotanteils R und der Intensität des Blauanteils B berechnet und mit einem vorgegebenen Grenzwert verglichen wird, wobei auf den Eintritt von Blut geschlossen wird, wenn der Quotient größer als der vorgegebene Grenzwert ist oder dass die Auswerteinheit (20) derart ausgebildet ist, dass der Quotient (R/B) aus der Intensität des Rotanteils R und der Intensität des Blauanteils B berechnet und mit einem vorgegebenen Grenzwert verglichen wird, wobei auf eine Hämolyse geschlossen wird, wenn der Quotient kleiner oder gleich dem vorgegebenen Grenzwert ist.

5. Vorrichtung nach Anspruch 1 oder 2, **dadurch gekennzeichnet, dass** die Mittel (2 1 A) zum Emittieren von Licht und die Mittel (21B) zum Empfangen von Licht als Mittel zum Emittieren und Empfangen von Licht ausgebildet sind, das einen Blauanteil und einen Grünanteil aufweist, und
dass die Auswerteinheit (20) derart ausgebildet ist, dass die Intensität I_{1/B} des Blauanteils des aus der Flüssigkeit austretenden Lichts und die Intensität I_{1/G} des Grünanteils des aus der Flüssigkeit austretenden Lichts bestimmt wird, und
dass die Auswerteinheit (20) derart ausgebildet ist, dass die Intensität I_{1/G} des Grünanteils G mit der Intensität I_{1/B} des Blauanteils B verglichen wird, wobei zwischen dem Eintritt von Blut in das Flüssigkeitssystem und einer Hämolyse auf der Grundlage des Vergleichs zwischen der Intensität des Grünanteils und der Intensität des Blauanteils unterschieden wird.

6. Vorrichtung nach Anspruch 5, **dadurch gekennzeichnet, dass** die Auswerteinheit (20) derart ausgebildet ist, dass der Quotient (G/B) aus der Intensität des Grünanteils und der Intensität des Blauanteils berechnet und mit einem vorgegebenen Grenzwert verglichen wird, wobei auf den Eintritt von Blut geschlossen wird, wenn der Quotient größer als der vorgegebene Grenzwert ist oder die Auswerteinheit (20) derart ausgebildet ist, dass der Quotient (G/B) aus der Intensität des Grünanteils und der Intensität des Blauanteils B berechnet und mit einem vorgegebenen Grenzwert verglichen wird, wobei auf eine Hämolyse geschlossen wird, wenn der Quotient kleiner oder gleich dem vorgegebenen Grenzwert ist.

7. Vorrichtung nach Anspruch 1 oder 2, **dadurch gekennzeichnet, dass** die Mittel (21A) zum Emittieren von Licht und die Mittel (21B) zum Empfangen von Licht als Mittel zum Emittieren und Empfangen von Licht ausgebildet sind, das einen Rotanteil, einen Grünanteil und einen Blauanteil aufweist, und
dass die Auswerteinheit (20) derart ausgebildet ist, dass die Intensität I_{1/R} des Rotanteils des aus der Flüssigkeit austretenden Lichts und die Intensität I_{1/G} des Grünanteils des aus der Flüssigkeit austretenden Lichts bestimmt wird, und
dass die Auswerteinheit (17) derart ausgebildet ist, dass in einem ersten Schritt die Intensität I_{1/R} des Rotanteils mit der Intensität I_{1/G} des Grünanteils G verglichen wird, wobei auf den Eintritt von Blut in das Flüssigkeitssystem oder eine Hämolyse auf der Grundlage des Vergleichs zwischen der Intensität des Grünanteils und der Intensität des Rotanteils geschlossen wird,
und dass die Auswerteinheit (20) derart ausgebildet ist, dass für den Fall, dass in dem ersten Schritt die Auswerteinheit auf den Eintritt von Blut oder eine Hämolyse geschlossen hat, in einem zweiten Schritt die Intensität I_{1/R} des Rotanteils mit der Intensität I_{1/B} des Blauanteils verglichen wird, wobei zwischen dem Eintritt von Blut in das Flüssigkeitssystem und einer Hämolyse auf der Grundlage des Vergleichs zwischen der Intensität des Rotanteils und der Intensität des Blauanteils unterschieden wird.

8. Vorrichtung nach Anspruch 7, **dadurch gekennzeichnet, dass** die Auswerteinheit (20) derart ausgebildet ist, dass in dem ersten Schritt der Quotient aus der Intensität I_{1/R} des Rotanteils und der Intensität I_{1/G} des Grünanteils G berechnet wird, wobei auf den Eintritt von Blut oder eine Hämolyse geschlossen wird, wenn der Quotient größer als ein vorgegebener erster Grenzwert ist, und
dass die Auswerteinheit (20) derart ausgebildet ist, dass in dem zweiten Schritt der Quotient aus der Intensität I_{1/R} des Rotanteils R und der Intensität I_{1/B} des Blauanteils B berechnet wird, wobei auf den Eintritt von Blut geschlossen wird, wenn der Quotient größer als ein vorgegebener zweiter Grenzwert ist oder auf eine Hämolyse geschlossen wird, wenn der Quotient kleiner oder gleich dem vorgegebenen zweiten Grenzwert ist.

9. Vorrichtung nach Anspruch 1 oder 2, **dadurch gekennzeichnet, dass** die Mittel (21A) zum Emittieren von Licht und die Mittel (21B) zum Empfangen von Licht als Mittel zum Emittieren und Empfangen von Licht ausgebildet sind, das einen Rotanteil, einen Grünanteil und einen Blauanteil aufweist, und
dass die Auswerteinheit (20) derart ausgebildet ist, dass die Intensität I_{1/R} des Rotanteils, die Intensität I_{1/G} des Grünanteils und die Intensität I_{1/B} des Blauanteils des aus der Flüssigkeit austretenden Lichts bestimmt wird, und
dass die Auswerteinheit (20) derart ausgebildet ist, dass in einem ersten Schritt die Intensität I_{1/R} des Rotanteils mit der Intensität I_{1/G} des Grünanteils G verglichen wird, wobei auf den Eintritt von Blut in das Flüssigkeitssystem oder eine Hämolyse auf der Grundlage des Vergleichs zwischen der Intensität des Grünanteils und der Intensität des Rotanteils geschlossen wird, und
dass die Auswerteinheit (20) derart ausgebildet ist, dass für den Fall, dass in dem ersten Schritt die Auswerteinheit auf den Eintritt von Blut oder eine Hämolyse geschlossen hat, in einem zweiten Schritt die Intensität I_{1/G} des Grünanteils mit der Intensität I_{1/B} des Blauanteils verglichen wird, wobei zwischen dem Eintritt von Blut in das Flüssigkeitssystem und einer Hämolyse auf der Grundlage des Vergleichs zwischen der Intensität des Grünanteils und der Intensität des Blauanteils unterschieden wird, wobei
die Auswerteinheit (20) derart ausgebildet ist, dass in dem ersten Schritt der Quotient aus der Intensität I_{R} des Rotanteils R und der Intensität I_{G} des Grünanteils G berechnet wird, wobei auf den Eintritt von Blut oder eine Hämolyse geschlossen wird, wenn der Quotient größer als ein vorgegebener erster Grenzwert ist, und
die Auswerteinheit (20) derart ausgebildet ist, dass in dem zweiten Schritt der Quotient aus der Intensität I_{1/G} des Grünanteils G und der Intensität I_{1/B} des Blauanteils B berechnet wird, wobei auf den Eintritt von Blut geschlossen wird, wenn der Quotient größer als ein vorgegebener zweiter Grenzwert ist oder auf eine Hämolyse geschlossen wird, wenn der Quotient kleiner oder gleich dem vorgegebenen zweiten Grenzwert ist.

10. Vorrichtung nach einem der Ansprüche 1 bis 9, **dadurch gekennzeichnet, dass** Mittel (21C) zum Empfangen von Licht, das in die im Flüssigkeitssystem befindliche Flüssigkeit eintritt, vorgesehen sind, wobei die Auswerteinheit (20) derart ausgebildet ist, dass die Intensität I₀ des in die Flüssigkeit eintretenden Lichts mit einem vorgegebenen Grenzwert verglichen wird.

11. Vorrichtung nach einem der Ansprüche 1 bis 10, **dadurch gekennzeichnet, dass** die Mittel (21, 21A) zum Emittieren von Licht eine Lichtquelle sind, die weißes Licht emittiert, wobei die Mittel (21, 21A, 21B) zum Empfangen von Licht ein Lichtsensor sind, der unterschiedliche Farbanteile des Lichts empfängt.

12. Vorrichtung zur extrakorporalen Blutbehandlung, die einen durch eine semipermeable Membran (2) in eine erste Kammer (3) und eine zweite Kammer (4) unterteilten Dialysator (1) oder Filter aufweist, wobei die erste Kammer (3) Teil eines extrakorporalen Blutkreislaufs (I) und die zweite Kammer (4) des Dialysators (1) oder Filters Teil eines Flüssigkeitssystems (II) ist, mit einer Vorrichtung zur Erkennung von Blut oder Blutbestandteilen im Flüssigkeitssystem nach einem der Ansprüche 1 bis 11.

13. Verfahren zur Erkennung von Blut oder Blutbestandteilen im Flüssigkeitssystem einer Vorrichtung zur extrakorporalen Blutbehandlung, die einen durch eine semipermeable Membran in eine erste Kammer und eine zweite Kammer unterteilten Dialysator oder Filter aufweist, wobei die erste Kammer Teil eines extrakorporalen Blutkreislaufs und die zweite Kammer Teil eines Flüssigkeitssystems ist, mit folgenden Verfahrenschritten:
Emittieren von Licht, das in die im Flüssigkeitssystem befindliche Flüssigkeit eintritt, und Empfangen von Licht, das aus der im Flüssigkeitssystem befindlichen Flüssigkeit austritt, und
Auswerten der Intensität I₁ des aus der Flüssigkeit austretenden Lichts,
**dadurch gekennzeichnet, dass**
auf der Grundlage der Änderung der Intensität I₁ des aus der Flüssigkeit austretenden Lichts zwischen dem Eintritt von Blut in das Flüssigkeitssystem aufgrund eines Defekts des Dialysators oder Filters oder des Eintritts von Hämoglobin in das Flüssigkeitssystem aufgrund einer Hämolyse unterschieden wird.

14. Verfahren nach Anspruch 13, **dadurch gekennzeichnet, dass** zwischen dem Eintritt von Blut in das Flüssigkeitssystem aufgrund eines Defekts des Dialysators oder Filters oder des Eintritts von Hämoglobin in das Flüssigkeitssystem aufgrund einer Hämolyse zumindest auf der Grundlage der Änderung der Intensität I₁ des Blauanteils des aus der Flüssigkeit austretenden Lichts unterschieden wird.

15. Verfahren nach Anspruch 13 oder 14, **dadurch gekennzeichnet, dass** Licht emittiert und empfangen wird, das zumindest einen Blauanteil und einen Rotanteil aufweist, und
dass die Intensität I_{1/B} des Blauanteils des aus der Flüssigkeit austretenden Lichts und die Intensität I_{1/R} des Rotanteils des aus der Flüssigkeit austretenden Lichts bestimmt wird, und
dass die Intensität I_{1/R} des Rotanteils mit der Intensität I_{1/B} des Blauanteils verglichen wird, wobei zwischen dem Eintritt von Blut in das Flüssigkeitssystem und einer Hämolyse auf der Grundlage des Vergleichs der Intensität des Rotanteils R mit der Intensität des Blauanteils B unterschieden wird.

16. Verfahren nach Anspruch 15, **dadurch gekennzeichnet, dass** der Quotient (R/B) aus der Intensität des Rotanteils und der Intensität des Blauanteils berechnet und mit einem vorgegebenen Grenzwert verglichen wird, wobei auf den Eintritt von Blut geschlossen wird, wenn der Quotient größer als der vorgegebene Grenzwert ist oder der Quotient (R/B) von der Intensität des Rotanteils und der Intensität des Blauanteils berechnet und mit einem vorgegebenen Grenzwert verglichen wird, wobei auf eine Hämolyse geschlossen wird, wenn der Quotient kleiner oder gleich dem vorgegebenen Grenzwert ist.

17. Verfahren nach Anspruch 13 oder 14, **dadurch gekennzeichnet, dass** Licht emittiert und empfangen wird, das zumindest einen Blauanteil und einen Grünanteil aufweist, und
dass die Intensität I_{1/B} des Blauanteils des aus der Flüssigkeit austretenden Lichts und die Intensität I_{1/G} des Grünanteils des aus der Flüssigkeit austretenden Lichts bestimmt wird, und
dass die Intensität des Grünanteils G mit der Intensität des Blauanteils B verglichen wird, wobei zwischen dem Eintritt von Blut in das Flüssigkeitssystem und einer Hämolyse auf der Grundlage des Vergleichs der Intensität des Grünanteils und der Intensität des Blauanteils B unterschieden wird, wobei
der Quotient (G/B) aus der Intensität des Grünanteils G und der Intensität des Blauanteils B berechnet und mit einem vorgegebenen Grenzwert verglichen wird, wobei auf einen Eintritt von Blut geschlossen wird, wenn der Quotient größer als der vorgegebene Grenzwert ist oder der Quotient (G/B) aus der Intensität des Grünanteils G und der Intensität des Blauanteils B berechnet und mit einem vorgegebenen Grenzwert verglichen wird, wobei auf eine Hämolyse geschlossen wird, wenn der Quotient kleiner oder gleich dem vorgegebenen Grenzwert ist.

18. Verfahren nach Anspruch 13 oder 14, **dadurch gekennzeichnet, dass** Licht emittiert und empfangen wird, dass einen Rotanteil, einen Grünanteil und einen Blauanteil aufweist, und
dass die Intensität I_{1/R} des Rotanteils, die Intensität I_{1/G} des Grünanteils und die Intensität I_{1/B} des Blauanteils des aus der Flüssigkeit austretenden Lichts bestimmt wird, und
dass in einem ersten Schritt die Intensität I_{1/R} des Rotanteils mit der Intensität I_{1/G} des Grünanteils G verglichen wird, wobei auf den Eintritt von Blut in das Flüssigkeitssystem oder eine Hämolyse auf der Grundlage des Vergleichs zwischen der Intensität des Rotanteils und der Intensität des Grünanteils geschlossen wird, und
dass für den Fall, dass in dem ersten Schritt auf den Eintritt von Blut oder eine Hämolyse geschlossen wird, in einem zweiten Schritt die Intensität I_{1/R} des Rotanteils mit der Intensität I_{1/B} des Blauanteils verglichen wird, wobei zwischen dem Eintritt von Blut in das Flüssigkeitssystem und einer Hämolyse auf der Grundlage des Vergleichs zwischen der Intensität des Rotanteils und der Intensität des Blauanteils unterschieden wird, wobei
in dem ersten Schritt der Quotient aus der Intensität I_{/1R} des Rotanteils und der Intensität I_{1/G} des Grünanteils G berechnet wird, wobei auf den Eintritt von Blut oder eine Hämolyse geschlossen wird, wenn der Quotient größer als ein vorgegebener erster Grenzwert ist, und
in dem zweiten Schritt der Quotient aus der Intensität I_{1/R} des Rotanteils und der Intensität I_{1/B} des Blauanteils B berechnet wird, wobei auf den Eintritt von Blut geschlossen wird, wenn der Quotient größer als ein vorgegebener zweiter Grenzwert ist, oder auf eine Hämolyse geschlossen wird, wenn der Quotient kleiner oder gleich dem vorgegebenen zweiten Grenzwert ist.

19. Verfahren nach Anspruch 13 oder 14, **dadurch gekennzeichnet, dass** Licht emittiert und empfangen wird, dass einen Rotanteil, einen Grünanteil und einen Blauanteil aufweist, und
dass die Intensität I_{1/R} des Rotanteils, die Intensität I_{1/G} des Grünanteils und die Intensität I_{1/B} des Blauanteils des aus der Flüssigkeit austretenden Lichts bestimmt wird, und
dass in einem ersten Schritt die Intensität I_{1/R} des Rotanteils mit der Intensität I_{1/G} des Grünanteils G verglichen wird, wobei auf den Eintritt von Blut in das Flüssigkeitssystem oder eine Hämolyse auf der Grundlage des Vergleichs zwischen der Intensität des Rotanteils und der Intensität des Grünanteils geschlossen wird, und
dass für den Fall, dass in dem ersten Schritt auf den Eintritt von Blut oder eine Hämolyse geschlossen wird, in einem zweiten Schritt die Intensität I_{1/B} des Grünanteils mit der Intensität I_{1/B} des Blauanteils verglichen wird, wobei zwischen dem Eintritt von Blut in das Flüssigkeitssystem und einer Hämolyse auf der Grundlage des Vergleichs zwischen der Intensität des Grünanteils und der Intensität des Blauanteils unterschieden wird, wobei in dem ersten Schritt der Quotient aus der Intensität I_{/1R} des Rotanteils und der Intensität I_{1/B} des Grünanteils G berechnet wird, wobei auf den Eintritt von Blut oder eine Hämolyse geschlossen wird, wenn der Quotient größer als ein vorgegebener erster Grenzwert ist, und in dem zweiten Schritt der Quotient aus der Intensität I_{1/G} des Grünanteils und der Intensität I_{1/B} des Blauanteils B berechnet wird, wobei auf den Eintritt von Blut geschlossen wird, wenn der Quotient größer als ein vorgegebener zweiter Grenzwert ist, oder auf eine Hämolyse geschlossen wird, wenn der Quotient kleiner oder gleich dem vorgegebenen zweiten Grenzwert ist.

20. Verfahren nach einem der Ansprüche 13 bis 19, **dadurch gekennzeichnet, dass** Licht, das in die im Flüssigkeitssystem befindliche Flüssigkeit eintritt, empfangen wird, wobei die Intensität I₀ des in die Flüssigkeit eintretenden Lichts mit einem vorgegebenen Grenzwert verglichen wird.

## Claims

1. A device for the detection of blood or blood constituents in the fluid system of an apparatus for extracorporeal blood treatment, which device comprises a dialyser (1) or filter divided by a semipermeable membrane (2) into a first chamber (3) and a second chamber (4), wherein the first chamber is part of the extracorporeal blood circuit (I) and the second chamber is part of a fluid system (II), with means (21A) for emitting light which enters into the fluid present in the fluid system, and means (2 1 B) for receiving light which exits from the fluid present in the fluid system, and
an evaluation unit (20) which comprises means for evaluating intensity I₁ of the light exiting from the fluid,
**characterised in that**
the device for the detection of blood or blood constituents in the fluid system is designed as a device for distinguishing between the entry of blood into the fluid system due to a defect in the dialyser or filter or the entry of haemoglobin into the fluid system due to a haemolysis on the basis of the change in intensity I₁ of the light exiting from the fluid.

2. The device according to claim 1, **characterised in that** the device for the detection of blood or blood constituents in the fluid system is designed such that a distinction is made between the entry of blood into the fluid due to a defect in the dialyser or filter or the entry of haemoglobin into the fluid system due to a haemolysis at least on the basis of the change in intensity I₁ of the blue component of the light exiting from the fluid.

3. The device according to claim 1 or 2, **characterised in that** the means (21) for emitting light and the means (21) for receiving light are designed as a means for emitting and receiving light which comprises at least a blue component and a red component, and
that the evaluation unit (20) is designed such that intensity I_{1/B} of the blue component of the light exiting from the fluid and intensity I_{1/R} of the red component of the light exiting from the fluid is determined, and
that the evaluation unit (20) is designed such that intensity I_{1/R} of the red component is compared with intensity I_{1/B} of the blue component, wherein a distinction is made between the entry of blood into the fluid system and a haemolysis on the basis of the comparison between the intensity of the red component and the intensity of the blue component.

4. The device according to claim 3, **characterised in that** the evaluation unit (20) is designed such that the quotient (R/B) of the intensity of red component R and the intensity of blue component B is calculated and compared with a preset threshold value, wherein it is concluded that there is an entry of blood if the quotient is greater than the preset threshold value or **in that** the evaluation unit (20) is designed such that the quotient (R/B) of the intensity of red component R and the intensity of blue component B is calculated and compared with a preset threshold value, wherein it is concluded that there is a haemolysis if the quotient is less than or equal to the preset threshold value.

5. The device according to claim 1 or 2, **characterised in that** the means (21 A) for emitting light and the means (2 1 B) for receiving light are designed as a means for emitting and receiving light which comprises a blue component and a green component, and
that the evaluation unit (20) is designed such that intensity I_{1/B} of the blue component of the light exiting from the fluid and intensity I_{1/G} of the green component of the light exiting from the fluid is determined, and
that the evaluation unit (20) is designed such that intensity I_{1/G} of green component G is compared with intensity I_{1/B} of blue component B, wherein a distinction is made between the entry of blood into the fluid system and a haemolysis on the basis of the comparison between the intensity of the green component and the intensity of the blue component.

6. The device according to claim 5, **characterised in that** the evaluation unit (20) is designed such that the quotient (G/B) of the intensity of the green component and intensity of the blue component is calculated and compared with a preset threshold value, wherein it is concluded that there is an entry of blood if the quotient is greater than the preset threshold value or the evaluation unit (20) is designed such that the quotient (G/B) of the intensity of the green component and the intensity of blue component B is calculated and compared with a preset threshold value, wherein it is concluded that there is a haemolysis if the quotient is less than or equal to the threshold value.

7. The device according to claim 1 or 2, **characterised in that** the means (21A) for emitting light and the means (21B) for receiving light are designed as a means for emitting and receiving light which comprises a red component, a green component and a blue component, and
that the evaluation unit (20) is designed such that intensity I_{1/R} of the red component of the light exiting from the fluid and intensity I_{1/G} of the green component of the light exiting from the fluid is determined, and
that the evaluation unit (17) is designed such that intensity I_{1/R} of the red component is compared with intensity I_{1/G} of green component G in a first step, wherein it is concluded that there is an entry of blood into the fluid system or a haemolysis
on the basis of the comparison between the intensity of the green component and intensity of the blue component,
and that the evaluation unit (20) is designed such that, in the event that the evaluation unit concludes that there is an entry of blood or a haemolysis in the first step, intensity I_{1/R} of the red component is compared with intensity I_{1/B} of the blue component in a second step, wherein a distinction is made between the entry of blood into the fluid system and a haemolysis on the basis of the comparison between the intensity of the red component and the intensity of the blue component.

8. The device according to claim 7, **characterised in that** the evaluation unit (20) is designed such that the quotient of intensity I_{1/R} of the red component and intensity I_{1/G} of green component G is calculated in the first step, wherein it is concluded that there is an entry of blood or a haemolysis if the quotient is greater than a preset first threshold value, and
that the evaluation unit (20) is designed such that the quotient of intensity I_{1/R} of red component R and intensity I_{1/B} of blue component B is calculated in the second step, wherein it is concluded that there is an entry of blood if the quotient is greater than a preset second threshold value or it is concluded that there is a haemolysis if the quotient is less than or equal to the preset second threshold value.

9. The device according to claim 1 or 2, **characterised in that** the means (21 A) for emitting light and the means (21 B) for receiving light are designed as a means for emitting and receiving light which comprises a red component, a green component and a blue component, and
that the evaluation unit (20) is designed such that intensity I_{1/R} of the red component, intensity I_{1/G} of the green component and intensity I_{1/B} of the blue component of the light exiting from the fluid is determined, and
that the evaluation unit (20) is designed such that
intensity I_{1/R} of the red component is compared with intensity I_{1/G} of green component G in a first step, wherein it is concluded that there is an entry of blood into the fluid system or a haemolysis on the basis of the comparison between the intensity of the green component and the intensity of the blue component, and that the evaluation unit (20) is designed such that, in the event that the evaluation unit concludes that there is an entry of blood or a haemolysis in the first step, intensity I_{1/G} of the green component is compared with intensity I_{1/B} of the blue component in a second step, wherein a distinction is made between the entry of blood into the fluid system and a haemolysis on the basis of the comparison between the intensity of the green component and the intensity of the blue component, wherein
the evaluation unit (20) is designed such that the quotient of intensity I_{R} of red component R and intensity I_{G} of green component G is calculated in the first step, wherein it is concluded that there is an entry of blood or a haemolysis if the quotient is greater than a preset first threshold value, and
that the evaluation unit (20) is designed such that the quotient of intensity I_{1/G} of green component G and intensity I_{1/B} of blue component B is calculated in the second step, wherein it is concluded that there is an entry of blood if the quotient is greater than a preset second threshold value or it is concluded that there is a haemolysis if the quotient is less than or equal to the preset second threshold value.

10. The device according to any one of claims 1 through 9, **characterised in that** means (21 C) are provided for receiving light which enters into the fluid present in the fluid system, wherein the evaluation unit (20) is designed such that intensity I₀ of the light entering into the fluid is compared with a preset threshold value.

11. The device according to any one of claims 1 through 10, **characterised in that** the means (21, 21A) for emitting light are a light source which emits white light, wherein the means (21, 21A, 21B) for receiving light are a light sensor which receives different colour components of light.

12. The device for extracorporeal blood treatment which comprises a dialyser (1) or filter divided by a semipermeable membrane (2) into a first chamber (3) and a second chamber (4), wherein the first chamber (3) is part of an extracorporeal blood circuit (I) and the second chamber (4) of the dialyser (1) or filter is part of a fluid system (II), with a device for the detection of blood or blood constituents in the fluid system according to any one of claims 1 through 11.

13. A method for the detection of blood or blood constituents in the fluid system of an apparatus for extracorporeal blood treatment which comprises a dialyser or filter divided by a semipermeable membrane into a first chamber and a second chamber, wherein the first chamber is part of an extracorporeal blood circuit and the second chamber is part of a fluid system, with the following process steps:
emitting of light which enters into the fluid present in the fluid system and
receiving of light which exits from the fluid present in the fluid system, and evaluation of intensity I₁ of the light exiting from the fluid,
**characterised in that**,
on the basis of the change in intensity I₁ of the light exiting from the fluid, a distinction is made between the entry of blood into the fluid system due to a defect in the dialyser or filter or the entry of haemoglobin into the fluid system due to a haemolysis.

14. The method according to claim 13, **characterised in that** a distinction is made between the entry of blood into the fluid system due to a defect in the dialyser or filter and the entry of haemoglobin into the fluid system due to a haemolysis at least on the basis of the change in intensity I₁ of the blue component of the light exiting from the fluid.

15. The method according to claim 13 or 14, **characterised in that** light is emitted and received, which light comprises at least a blue component and a red component, and
that intensity I_{1/B} of the blue component of the light exiting from the fluid and intensity I_{1/R} of the red component of the light exiting from the fluid is determined, and
that intensity I_{1/R} of the red component is compared with intensity I_{1/B} of the blue component, wherein a distinction is made between the entry of blood into the fluid system and a haemolysis on the basis of the comparison between the intensity of red component R and the intensity of blue component B.

16. The method according to claim 15, **characterised in that** the quotient (R/B) of the intensity of the red component and the intensity of the blue component is calculated and compared with a preset threshold value, wherein it is concluded that there is an entry of blood if the quotient is greater than the preset threshold value or the quotient (R/B) of the intensity of the red component and the intensity of the blue component is calculated and compared with a preset threshold value, wherein it is concluded that there is a haemolysis if the quotient is less than or equal to the preset threshold value.

17. The method according to claim 13 or 14, **characterised in that** light is emitted and received which comprises at least a blue component and a green component, and that intensity I_{1/B} of the blue component of the light exiting from the fluid and intensity I_{1/G} of the green component of the light exiting from the fluid is determined, and
that the intensity of green component G is compared with the intensity of blue component B,
wherein a distinction is made between the entry of blood into the fluid system and a haemolysis on the basis of the comparison of the intensity of the green component and the intensity of blue component B, wherein
the quotient (G/B) of the intensity of green component G and the intensity of blue component B is calculated and compared with a preset threshold value, wherein it is concluded that there is an entry of blood if the quotient is greater than the preset threshold value, or the quotient (G/B) of the intensity of green component G and the intensity of blue component B is calculated and compared with a preset threshold value, wherein it is concluded that there is a haemolysis if the quotient is less than or equal to the preset threshold value.

18. The method according to claim 13 or 14, **characterised in that** light is emitted and received which comprises a red component, a green component and a blue component, and
that intensity I_{1/R} of the red component, intensity I_{1/G} of the green component and intensity I_{1/B} of the blue component of the light exiting from the fluid is determined, and
that intensity I_{1/R} of the red component is compared with intensity I_{1/G} of green component G in a first step, wherein it is concluded that there is an entry of blood into the fluid system or a haemolysis on the basis of the comparison between the intensity of the red component and the intensity of the green component, and that, in the event that it is concluded that there is an entry of blood or a haemolysis in the first step, intensity I_{1/R} of the red component is compared with intensity I_{1/B} of the blue component in a second step, wherein a distinction is made between the entry of blood into the fluid system and a haemolysis on the basis of the comparison between the intensity of the red component and the intensity of the blue component, wherein
the quotient of intensity I_{1/R} of the red component and
intensity I_{1/G} of green component G is calculated in the first step, wherein it is concluded that there is an entry of blood or a haemolysis if the quotient is greater than a preset first threshold value, and
the quotient of intensity I_{1/R} of the red component and intensity I_{1/B} of blue component B is calculated in the second step, wherein it is concluded that there is an entry of blood if the quotient is greater than a preset second threshold value or it is concluded that there is a haemolysis if the quotient is less than or equal to the preset second threshold value.

19. The method according to claim 13 or 14, **characterised in that** light is emitted and received which comprises a red component, a green component and a blue component, and
that intensity I_{1/R} of the red component, intensity I_{1/G} of the green component and intensity I_{1/B} of the blue component of the light exiting from the fluid is determined, and
that intensity I_{1/R} of the red component is compared with intensity I_{1/G} of green component G in a first step, wherein it is concluded that there is an entry of blood into the fluid system or a haemolysis on the basis of the comparison between the intensity of the red component and the intensity of the green component, and that, in the event that it is concluded that there is an entry of blood or a haemolysis in the first step, intensity I_{1/G} of the green component is compared with intensity I_{1/B} of the blue component in a second step, wherein a distinction is made between the entry of blood into the fluid system and a haemolysis on the basis of the comparison between the intensity of the green component and intensity of the blue component, wherein the quotient of intensity I_{1/R} of the red component and intensity I_{1/G} of green component G is calculated in the first step, wherein it is concluded that there is an entry of blood or a haemolysis if the quotient is greater than a preset first threshold value, and the quotient of intensity I_{1/G} of the green component and
intensity I_{1/B} of blue component B is calculated in the second step, wherein it is concluded that there is an entry of blood if the quotient is greater than a preset second threshold value or it is concluded that there is a haemolysis if the quotient is less than or equal to the preset second threshold value.

20. The method according to any one of claims 13 through 19, **characterised in that** light that enters into the fluid present in the fluid system is received, wherein intensity I₀ of the light entering into the fluid is compared with a preset threshold value.

## Revendications

1. Dispositif pour détecter du sang ou des constituants sanguins dans le système de liquide d'un dispositif de traitement extracorporel du sang, qui présente un dialyseur (1) ou filtre subdivisé par une membrane semi-perméable (2) en une première chambre (3) et une deuxième chambre (4), dans lequel la première chambre fait partie d'un circuit sanguin extracorporel (I) et la deuxième chambre fait partie d'un système de liquide (II), comportant
des moyens (21A) pour émettre de la lumière qui entre dans le liquide se trouvant dans le système de liquide et des moyens (21B) pour recevoir la lumière qui sort du liquide se trouvant dans le système de liquide, et
une unité d'analyse (20) qui présente des moyens pour analyser l'intensité I₁ de la lumière sortant du liquide,
**caractérisé en ce que**
le dispositif pour détecter du sang ou des constituants sanguins dans le système de liquide est conçu comme un appareil pour établir une distinction entre l'entrée de sang dans le système de liquide en raison d'un défaut du dialyseur ou du filtre ou de l'entrée d'hémoglobine dans le système de liquide en raison d'une hémolyse sur la base de la modification de l'intensité I₁ de la lumière sortant du liquide.

2. Dispositif selon la revendication 1, **caractérisé en ce que** l'appareil pour identifier du sang ou des constituants sanguins dans le système de liquide est conçu de telle sorte qu'entre l'entrée de sang dans le système de liquide en raison d'un défaut du dialyseur ou du filtre ou de l'entrée d'hémoglobine dans le système de liquide en raison d'une hémolyse, est établie une distinction au moins sur la base de la modification de l'intensité I₁ de la partie bleue de la lumière sortant du liquide.

3. Dispositif selon la revendication 1 ou 2, **caractérisé en ce que** les moyens (21) pour émettre de la lumière et les moyens (21) pour recevoir de la lumière sont conçus comme des moyens pour émettre et recevoir de la lumière, qui présente au moins une partie bleue et une partie rouge, et
**en ce que** l'unité d'analyse (20) est conçue de telle sorte que l'intensité I_{1/B} de la partie bleue de la lumière sortant du liquide et l'intensité I_{1/R} de la partie rouge de la lumière sortant du liquide soient déterminées, et
**en ce que** l'unité d'analyse (20) est conçue de telle sorte que l'intensité I_{1/R} de la partie rouge soit comparée à l'intensité I_{1/B} de la partie bleue, dans lequel une distinction est établie entre l'entrée de sang dans le système de liquide et une hémolyse sur la base de la comparaison entre l'intensité de la partie rouge et l'intensité de la partie bleue.

4. Dispositif selon la revendication 3, **caractérisé en ce que** l'unité d'analyse (20) est conçue de telle sorte que le quotient (R/B) soit calculé à partir de l'intensité de la partie rouge R et de l'intensité de la partie bleue B et soit comparé à une valeur seuil prédéterminée, dans lequel on conclut à l'entrée de sang lorsque le quotient est supérieur à la valeur seuil prédéterminée ou **en ce que** l'unité d'analyse (20) est conçue de telle sorte que le quotient (R/B) soit calculé à partir de l'intensité de la partie rouge R et de l'intensité de la partie bleue B et soit comparé à une valeur seuil prédéterminée, dans lequel on conclut à une hémolyse lorsque le quotient est inférieur ou égal à la valeur seuil prédéterminée.

5. Dispositif selon la revendication 1 ou 2, **caractérisé en ce que** les moyens (21A) pour émettre de la lumière et les moyens (21B) pour recevoir de la lumière sont conçus comme des moyens pour émettre et recevoir de la lumière, qui présente une partie bleue et une partie verte, et
**en ce que** l'unité d'analyse (20) est conçue de telle sorte que l'intensité I_{1/B} de la partie bleue de la lumière sortant du liquide et l'intensité I_{1/G} de la partie verte de la lumière sortant du liquide soient déterminées, et
**en ce que** l'unité d'analyse (20) est conçue de telle sorte que l'intensité I_{1/G} de la partie verte G est comparée à l'intensité I_{1/B} de la partie bleue B, dans lequel une distinction est établie entre l'entrée de sang dans le système de liquide et une hémolyse sur la base de la comparaison entre l'intensité de la partie verte et l'intensité de la partie bleue.

6. Dispositif selon la revendication 5, **caractérisé en ce que** l'unité d'analyse (20) est conçue de telle sorte que le quotient (G/B) soit calculé à partir de l'intensité de la partie verte et de l'intensité de la partie bleue et soit comparé à une valeur seuil prédéterminée, dans lequel on conclut à l'entrée de sang lorsque le quotient est supérieur à la valeur seuil prédéterminée ou **en ce que** l'unité d'analyse (20) est conçue de telle sorte que le quotient (G/B) soit calculé à partir de l'intensité de la partie verte et de l'intensité de la partie bleue B et soit comparé à une valeur seuil prédéterminée, dans lequel on conclut à une hémolyse lorsque le quotient est inférieur ou égal à la valeur seuil prédéterminée.

7. Dispositif selon la revendication 1 ou 2, **caractérisé en ce que** les moyens (21A) pour émettre de la lumière et les moyens (21B) pour recevoir de la lumière sont conçus comme des moyens pour émettre et recevoir de la lumière, qui présente une partie rouge, une partie verte et une partie bleue, et
**en ce que** l'unité d'analyse (20) est conçue de telle sorte que l'intensité I_{1/R} de la partie rouge de la lumière sortant du liquide et l'intensité I_{1/G} de la partie verte de la lumière sortant du liquide soient déterminées, et
**en ce que** l'unité d'analyse (17) est conçue de telle sorte que dans une première étape, l'intensité I_{1/R} de la partie rouge soit comparée à l'intensité I_{1/G} de la partie verte G, dans lequel on conclut à l'entrée de sang dans le système de liquide ou à une hémolyse sur la base de la comparaison entre l'intensité de la partie verte et l'intensité de la partie rouge,
et **en ce que** l'unité d'analyse (20) est conçue de telle sorte que dans le cas où, dans la première étape, l'unité d'analyse a conclu à l'entrée de sang ou à une hémolyse, dans une deuxième étape, l'intensité I_{1/R} de la partie rouge est comparée à l'intensité I_{1/E} de la partie bleue, dans lequel une distinction est établie entre l'entrée de sang dans le système de liquide et une hémolyse sur la base de la comparaison entre l'intensité de la partie rouge et l'intensité de la partie bleue.

8. Dispositif selon la revendication 7, **caractérisé en ce que** l'unité d'analyse (20) est conçue de telle sorte que dans la première étape, le quotient soit calculé à partir de l'intensité I_{1/R} de la partie rouge et de l'intensité I_{1/G} de la partie verte G, dans lequel on conclut à l'entrée de sang ou à une hémolyse lorsque le quotient est supérieur à une première valeur seuil prédéterminée, et
**en ce que** l'unité d'analyse (20) est conçue de telle sorte que dans la deuxième étape, le quotient soit calculé à partir de l'intensité I_{1/R} de la partie rouge R et de l'intensité I_{1/B} de la partie bleue B, dans lequel on conclut à l'entrée de sang lorsque le quotient est supérieur à une deuxième valeur seuil prédéterminée ou à une hémolyse lorsque le quotient est inférieur ou égal à la deuxième valeur seuil prédéterminée.

9. Dispositif selon la revendication 1 ou 2, **caractérisé en ce que** les moyens (21A) pour émettre de la lumière et les moyens (21B) pour recevoir de la lumière sont conçus comme des moyens pour émettre et recevoir de la lumière, qui présente une partie rouge, une partie verte et une partie bleue, et
**en ce que** l'unité d'analyse (20) est conçue de telle sorte que l'intensité I_{1/R} de la partie rouge, l'intensité I_{1/G} de la partie verte et l'intensité I_{1/B} de la partie bleue soient déterminées à partir de la lumière sortant du liquide, et
**en ce que** l'unité d'analyse (20) est conçue de telle sorte que dans une première étape, l'intensité I_{1/R} de la partie rouge soit comparée à l'intensité I_{1/G} de la partie verte, dans lequel on conclut à l'entrée de sang dans le système de liquide ou à une hémolyse sur la base de la comparaison entre l'intensité de la partie verte et l'intensité de la partie rouge, et **en ce que** l'unité d'analyse (20) est conçue de telle sorte que dans le cas où, dans la première étape, l'unité d'analyse a conclu à l'entrée de sang ou à une hémolyse, dans une deuxième étape, l'intensité I_{1/G} de la partie verte soit comparée à l'intensité I_{1/B} de la partie bleue, dans lequel une distinction est établie entre l'entrée de sang dans le système de liquide et une hémolyse sur la base de la comparaison entre l'intensité de la partie verte et l'intensité de la partie bleue, dans lequel
l'unité d'analyse (20) est conçue de telle sorte que dans la première étape, le quotient soit calculé à partir de l'intensité I_{R} de la partie rouge R et de l'intensité I_{G} de la partie verte G, dans lequel on conclut à l'entrée de sang ou à une hémolyse lorsque le quotient est supérieur à une première valeur seuil prédéterminée, et
l'unité d'analyse (20) est conçue de telle sorte que dans la deuxième étape, le quotient soit calculé à partir de l'intensité I_{1/G} de la partie verte G et de l'intensité U_{1/B} de la partie bleue B, dans lequel on conclut à l'entrée de sang lorsque le quotient est supérieur à une deuxième valeur seuil prédéterminée ou à une hémolyse lorsque le quotient est inférieur ou égal à la deuxième valeur seuil prédéterminée.

10. Dispositif selon l'une des revendications 1 à 9, **caractérisé en ce que** des moyens (21 C) pour recevoir de la lumière qui entre dans le liquide se trouvant dans le système de liquide sont prévus, dans lequel l'unité d'analyse (20) est conçue de telle sorte que l'intensité I₀ de la lumière entrant dans le liquide soit comparée à une valeur seuil prédéterminée.

11. Dispositif selon l'une des revendications 1 à 10, **caractérisé en ce que** les moyens (21, 21A) pour émettre de la lumière sont une source de lumière qui émet une lumière blanche, dans lequel les moyens (21, 21A, 21B) pour recevoir de la lumière sont un capteur de lumière qui reçoit des composantes chromatiques différentes de la lumière.

12. Dispositif pour le traitement extracorporel du sang, qui présente un dialyseur (1) ou filtre subdivisé par une membrane semi-perméable (2) en une première chambre (3) et une deuxième chambre (4), dans lequel la première chambre (3) fait partie d'un circuit sanguin extracorporel (I) et la deuxième chambre (4) du dialyseur (1) ou du filtre fait partie d'un système de liquide (II), comportant un dispositif pour détecter du sang ou des constituants sanguins dans le système de liquide selon l'une des revendications 1 à 11.

13. Procédé pour détecter du sang ou des constituants sanguins dans le système de liquide d'un dispositif de traitement extracorporel du sang, qui présente un dialyseur ou filtre subdivisé par une membrane semi-perméable en une première chambre et une deuxième chambre, dans lequel la première chambre fait partie d'un circuit sanguin extracorporel et la deuxième chambre fait partie d'un système de liquide, comportant les étapes de procédé suivantes :
l'émission de lumière qui entre dans le liquide se trouvant dans le système de liquide et la réception de lumière qui sort du liquide se trouvant dans le système de liquide, et l'analyse de l'intensité I₁ de la lumière sortant du liquide,
**caractérisé en ce que**
une distinction est établie sur la base de la modification de l'intensité I₁ de la lumière sortant du liquide entre l'entrée de sang dans le système de liquide en raison d'un défaut du dialyseur ou du filtre ou de l'entrée d'hémoglobine dans le système de liquide en raison d'une hémolyse.

14. Procédé selon la revendication 13, **caractérisé en ce qu'**une distinction est établie entre l'entrée de sang dans le système de liquide en raison d'un défaut du dialyseur ou du filtre ou de l'entrée d'hémoglobine dans le système de liquide en raison d'une hémolyse au moins sur la base de la modification de l'intensité I₁ de la partie bleue de la lumière sortant du liquide.

15. Procédé selon la revendication 13 ou 14, **caractérisé en ce que** de la lumière est émise et reçue, laquelle présente au moins une partie bleue et une partie rouge, et
**en ce que** l'intensité I_{1/B} de la partie bleue de la lumière sortant du liquide et l'intensité I_{1/R} de la partie rouge de la lumière sortant du liquide sont déterminées, et
**en ce que** l'intensité I_{1/R} de la partie rouge est comparée à l'intensité I_{1/B} de la partie bleue, dans lequel une distinction est établie entre l'entrée de sang dans le système de liquide et une hémolyse sur la base de la comparaison de l'intensité de la partie rouge R à l'intensité de la partie bleue B.

16. Procédé selon la revendication 15, **caractérisé en ce que** le quotient (R/B) est calculé à partir de l'intensité de la partie rouge et de l'intensité de la partie bleue et est comparé à une valeur seuil prédéterminée, dans lequel on conclut à l'entrée de sang lorsque le quotient est supérieur à la valeur seuil prédéterminée ou le quotient (R/B) est calculé à partir de l'intensité de la partie rouge et de l'intensité de la partie bleue et est comparé à une valeur seuil prédéterminée, dans lequel on conclut à une hémolyse lorsque le quotient est inférieur ou égal à la valeur seuil prédéterminée.

17. Procédé selon la revendication 13 ou 14, **caractérisé en ce que** de la lumière est émise et reçue, laquelle présente au moins une partie bleue et une partie verte, et
**en ce que**
l'intensité I_{1/B} de la partie bleue de la lumière sortant du liquide et l'intensité I_{1/G} de la partie verte de la lumière sortant du liquide sont déterminées, et
**en ce que** l'intensité de la partie verte G est comparée à l'intensité de la partie bleue B, dans lequel une distinction est établie entre l'entrée de sang dans le système de liquide et une hémolyse sur la base de la comparaison de l'intensité de la partie verte et de l'intensité de la partie bleue B, dans lequel
le quotient (G/B) est calculé à partir de l'intensité de la partie verte G et de l'intensité de la partie bleue B et est comparé à une valeur seuil prédéterminée, dans lequel on conclut à une entrée de sang lorsque le quotient est supérieur à la valeur seuil prédéterminée ou le quotient (G/B) est calculé à partir de l'intensité de la partie verte G et de l'intensité de la partie bleue B et est comparé à une valeur seuil prédéterminée, dans lequel on conclut à une hémolyse lorsque le quotient est inférieur ou égal à la valeur seuil prédéterminée.

18. Procédé selon la revendication 13 ou 14, **caractérisé en ce que** de la lumière est émise et reçue, laquelle présente une partie rouge, une partie verte et une partie bleue, et
**en ce que** l'intensité I_{1/R} de la partie rouge, l'intensité I_{1/G} de la partie verte et l'intensité I_{1/B} de la partie bleue de la lumière sortant du liquide sont déterminées, et
**en ce que**, dans une première étape, l'intensité I_{1/R} de la partie rouge est comparée à l'intensité I_{1/G} de la partie verte G, dans lequel on conclut à l'entrée de sang dans le système de liquide ou à une hémolyse sur la base de la comparaison entre l'intensité de la partie rouge et l'intensité de la partie verte, et
**en ce que** dans le cas où, dans la première étape, on conclut à l'entrée de sang ou à une hémolyse, dans une deuxième étape, l'intensité I_{1/R} de la partie rouge est comparée à l'intensité I_{1/B} de la partie bleue, dans lequel une distinction est établie entre l'entrée de sang dans le système de liquide et une hémolyse sur la base de la comparaison entre l'intensité de la partie rouge et l'intensité de la partie bleue, dans lequel
dans la première étape, le quotient est calculé à partir de l'intensité I_{1/R} de la partie rouge et de l'intensité I_{1/G} de la partie verte G, dans lequel on conclut à l'entrée de sang ou à une hémolyse lorsque le quotient est supérieur à une première valeur seuil prédéterminée, et dans la deuxième étape, le quotient est calculé à partir de l'intensité I_{1/R} de la partie rouge et de l'intensité I_{1/B} de la partie bleue B, dans lequel on conclut à l'entrée de sang lorsque le quotient est supérieur à une deuxième valeur seuil prédéterminée ou à une hémolyse lorsque le quotient est inférieur ou égal à la deuxième valeur seuil prédéterminée.

19. Procédé selon la revendication 13 ou 14, **caractérisé en ce que** de la lumière est émise et reçue, laquelle présente une partie rouge, une partie verte et une partie bleue, et
**en ce que** l'intensité I_{1/R} de la partie rouge, l'intensité I_{1/G} de la partie verte et l'intensité I_{1/B} de la partie bleue de la lumière sortant du liquide sont déterminées, et
**en ce que**, dans une première étape, l'intensité I_{1/R} de la partie rouge est comparée à l'intensité I_{1/G} de la partie verte G, dans lequel on conclut à l'entrée de sang dans le système de liquide ou à une hémolyse sur la base de la comparaison entre l'intensité de la partie rouge et l'intensité de la partie verte, et
**en ce que** dans le cas où, dans la première étape, on conclut à l'entrée de sang ou à une hémolyse, dans une deuxième étape, l'intensité I_{1/G} de la partie verte est comparée à l'intensité I_{1/B} de la partie bleue, dans lequel une distinction est établie entre l'entrée de sang dans le système de liquide et une hémolyse sur la base de la comparaison entre l'intensité de la partie verte et l'intensité de la partie bleue, dans lequel dans la première étape, le quotient est calculé à partir de l'intensité I_{1/R} de la partie rouge et de l'intensité I_{1/G} de la partie verte G, dans lequel on conclut à l'entrée de sang ou à une hémolyse lorsque le quotient est supérieur à une première valeur seuil prédéterminée et, dans la deuxième étape, le quotient est calculé à partir de l'intensité I_{1/G} de la partie verte et de l'intensité I_{1/3} de la partie bleue B, dans lequel on conclut à l'entrée de sang lorsque le quotient est supérieur à une deuxième valeur seuil prédéterminée ou à une hémolyse lorsque le quotient est inférieur ou égal à la deuxième valeur seuil prédéterminée.

20. Procédé selon l'une des revendications 13 à 19, **caractérisé en ce que** de la lumière qui entre dans le liquide se trouvant dans le système de liquide est reçue, dans lequel l'intensité I₀ de la lumière entrant dans le liquide est comparée à une valeur seuil prédéterminée.
